# FASCICULE DE BREVET EUROPEEN

(11) **EP 0 629 635 B1**
(45) Date de publication et mention de la délivrance du brevet: **19.03.1997**
(21) Numéro de dépôt: 94401344.0
(22) Date de dépôt: 16.06.1994
(51) Int. Cl.: C07J 41/00, A61K 31/565, C07J 43/00, A61K 31/58

(54) **Nouveaux 19-Nor stéroides ayant en position 11bêta une chaîne phénoxyalkylsulfonamide ou phénoxyalkylsulfonylurée, procédé et intermédiaires de préparation, application comme médicaments et compositions pharmaceutiques les contenant**
Neue 19-Nor-Steroide mit einer 11-Beta-Phenoxysulfonamid- oder 11-Beta-Phenoxysulfonylurea-Gruppe, Verfahren und Zwischenprodukte zu ihrer Herstellung sowie Medikamenten und pharmazeutische Präparate davon
New 19-nor 11-beta-phenoxysulfonamide or 11-beta-phenoxyalkylsulfonylurea steriod derivatives, a process and intermediates for their production and their use as medicaments and pharmaceuticals containing them

(30) Priorité: 17.06.1993 FR 9307310
(43) Date de publication de la demande: 21.12.1994
(73) Titulaire: ROUSSEL UCLAF, 93230 Romainville (FR)
(72) Inventeur: Nique, François, F-94170 Le Perreux Sur Marne (FR); Teutsch, Jean-Georges, F-93500 Pantin (FR); Van De Velde, Patrick, F-75019 Paris (FR)
(74) Mandataire: Vieillefosse, Jean-Claude

(56) Documents cités:
- EP-A- 0 097 572
- EP-A- 0 384 842
- EP-A- 0 471 612
- WO-A-93/13123
- FR-A- 2 640 977
- EUROPEAN JOURNAL OF BIOCHEMISTRY, vol.199, no.3, 1991 pages 575 - 585 J. L. BORGNA ET AL 'Differential Interactions of Estrogens and Antiestrogens at The 17- -Hydroxy or Counterpart Function with The Estrogen Receptor'

## Description

La présente invention concerne de nouveaux 19-Nor stéroïdes ayant en position llbéta une chaîne phénoxyalkylsulfonamide ou phénoxyalkylsulfonylurée, leur procédé de préparation et les intermédiaires de ce procédé, leur application comme médicaments et les compositions pharmaceutiques les contenant.

Dans les demandes de brevets européens EP-A-384.482 et EP-A-471.612 sont décrits des stéroïdes substitués en position 11 par un groupement phényloxy-N,N-dialkylalkylamide. Les produits selon l'invention diffèrent des produits de l'art antérieur le plus proche, en ce qu'ils comportent une fonction sulfonamide ou sulfonylurée à la place de la fonction amide.

L'invention a pour objet les composés de formule (I) : 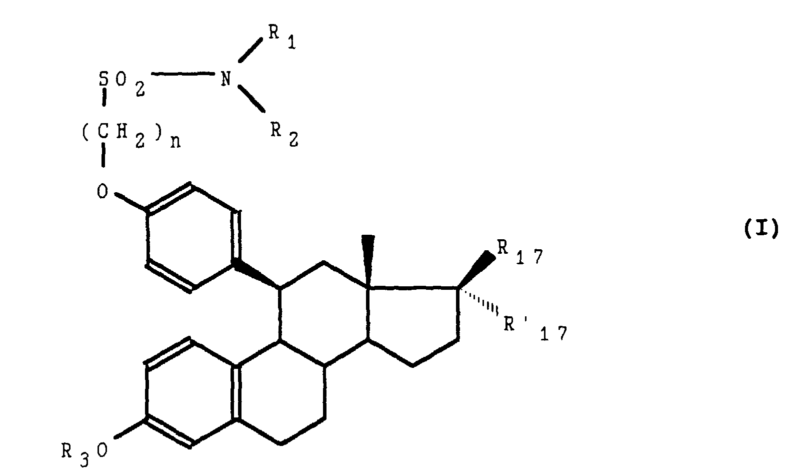 dans laquelle R₁₇ et R'₁₇ sont tels que :
- soit R₁₇ et R'₁₇ forment ensemble une cétone
- soit R₁₇ représente un radical hydroxyle, ou un radical acyloxy ayant au plus 12 atomes de carbone et R'₁₇ représente un atome d'hydrogène, un radical alkyle, alcényle ou alcynyle ayant au plus 8 atomes de carbone, chacun de ces radicaux étant éventuellement substitué,
- R₃ représente un atome d'hydrogène, un radical alkyle linéaire, ramifié ou cyclique ayant au plus 8 atomes de carbone ou un radical acyle ayant au plus 12 atomes de carbone,
et dans laquelle, R₁ et R₂ identiques ou différents représentent un atome d'hydrogène, un radical alkyle linéaire, ramifié ou cyclique renfermant de 1 à 8 atomes de carbone, éventuellement substitué, un radical acyle ayant au plus 12 atomes de carbone, un radical aryle ou aralkyle, chacun de ces radicaux étant éventuellement substitué, dans lesquels le radical alkyle renferme au plus 6 atomes de carbone et le radical aryle représente un radical mono ou polycyclique pouvant renfermer un ou plusieurs hétéroatomes choisis parmi les atomes d'oxygène, d'azote et de soufre,
- ou R₁ représente un radical carbamoyle monosubstitué par un radical alkyle linéaire, ramifié ou cyclique ayant au plus 8 atomes de carbone, éventuellement substitué, ou par un radical aryle ou aralkyle, chacun de ces radicaux étant éventuellement substitué, dans lesquels le radical alkyle renferme au plus 6 atomes de carbone et le radical aryle représente un radical mono ou polycyclique pouvant renfermer un ou plusieurs hétéroatomes choisis parmi les atomes d'oxygène, d'azote et de soufre et R₂ représente un atome d'hydrogène,
- ou R₁ et R₂ forment avec l'atome d'azote auquel ils sont liés un hétérocycle azoté saturé à 5 ou 6 chainons renfermant éventuellement un second hétéroatome, choisi parmi les atomes d'azote, d'oxygène et de soufre, éventuellement substitué par un radical alkyle ayant de 1 à 4 atomes de carbone, ou par un groupe oxo,
- ou R₁ et R₂ forment un radical dialkylaminométhylène, chaque alkyle renfermant de 1 à 4 atomes de carbone,
n est un entier égal au plus à 18, ainsi que les sels d'addition de ceux-ci.

Par radical acyloxy, il peut s'agir notamment du radical correspondant à un acide aliphatique ou cycloaliphatique saturé ou insaturé et notamment
d'un acide alcanoïque tel que par exemple l'acide acétique, propionique, butyrique ou isobutyrique, valérique ou undécylique,
d'un acide hydroxyalcanoïque tel que par exemple l'acide hydroxyacétique,
d'un acide cycloalkylcarboxylique ou cycloalkylalcanoïque tel que par exemple l'acide cyclopropyl, cyclopentyl ou cyclohexylcarboxylique, cyclopentyle ou cyclohexyle acétique ou propionique, d'un acide benzoïque, d'un acide salicylique ou d'un acide phénylalcanoïque tel que l'acide phényle acétique ou phényle propionique, d'un aminoacide tel que l'acide diéthylamino acétique ou aspartique, de l'acide formique ou d'un diacide éventuellement salifié, tel que par exemple l'acide butanedioïque ou le sel monosodique de celui-ci. Il s'agit de préférence du dérivé de l'acide acétique, propionique ou butyrique.

Par radical acyle, on entend les radicaux correspondants aux radicaux acyloxy précédents.

Par radical alkyle, il peut s'agir d'un radical méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle. Il s'agit de préférence du radical méthyle, éthyle, propyle, butyle.

Par radical cycloalkyle il peut s'agir d'un radical cyclopropyle, cyclobutyle, cyclopentyle ou cyclohexyle. Il s'agit de préférence du radical cyclopentyle.

Lorsque R'₁₇ représente un radical alcényle, il peut s'agir d'un radical vinyle, propényle, isopropényle, allyle, 2-méthylallyle, butényle ou isobutényle. Il s'agit de préférence du radical vinyle ou propényle.

Lorsque R'₁₇ représente un radical alcynyle, il peut s'agir du radical éthynyle, propynyle, propargyle, butynyle ou isobutynyle. Il s'agit de préférence du radical éthynyle ou propynyle.

Par radical aryle mono ou polycyclique, qui peut être compris dans un radical aralkyle, on entend :
- un radical monocyclique carbocyclique, par exemple le radical phényle,
- un radical monocyclique hétérocyclique, par exemple les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle, ainsi que les isomères de position du ou des hétéroatomes que ces radicaux peuvent renfermer,
- un radical constitué de cycles condensés carbocycliques, par exemple le radical naphtyle ou le radical phénanthrényle,
- un radical constitué de cycles condensés hétérocycliques, par exemple le benzofurannyle, le benzothiényle, le benzimidazolyle, le benzothiazolyle, le naphto[2,3-b]thiényle, le thianthrényle, l'isobenzofurannyle, le chroményle, le xanthényle, le phénoxathiinyle, l'indolizinyle, l'isoïndolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle,le phénazinyle, le phénothiazinyle, le phénoxazinyle, l'indolinyle, l'isoïndolinyle, l'imidazopyridyle, l'imidazopyrimidinyle ou encore les systèmes polycycliques condensés constitués de monocycliques hétérocycliques tels que définis, par exemple, ci-dessus comme par exemple le furo[2,3-b]pyrrole ou le thiéno[2,3-b]furanne, et plus particulièrement, les radicaux phényle, furyle tel que 2-furyle, imidazolyle tel que 2-imidazolyle, pyridyle tel que 2-pyridyle, 3-pyridyle, 4-pyridyle, pyrimidinyle tel que pyrimid-2-yle, thiazolyle tel que thiazol-2-yle, thiazolinyle tel que thiazolin-2-yle, triazolyle tel que triazol-2-yle, tétrazolyle tel que tétrazol-2-yle, benzimidazolyle tel que benzimidazol-2-yle, benzothiazolyle tel que benzothiazol-2-yle, purinyle tel que purin-7-yle ou quinolyle tel que 4-quinolyle, et comme exemple de radicaux aralkyle, on peut citer en particulier les radicaux méthyle ou éthyle substitués par l'un des radicaux aryles ci-dessus.

Par hétérocycle azoté saturé à 5 ou 6 chainons, renfermant éventuellement un second hétéroatome, choisi parmi les atomes d'azote, d'oxygène et de soufre et éventuellement substitué par un radical alkyle ou par un groupe carbonyle, il s'agit de préférence de la pyrrolidine, de la pipéridine, de la pipérazine, de la morpholine, de la thiamorpholine ou de l'imidazolidinone.

Les substituants des différents radicaux énoncés plus haut sont choisis de préférence dans le groupe constitué par :
- halogène : fluor, chlore, brome, iode,
- amino, alkylamino tel que méthylamino ou éthylamino, dialkylamino tel que diméthylamino, diéthylamino, méthyléthylamino, chacun de ces radicaux dialkylamino étant éventuellement sous forme oxydée,
- aminoalkyle tel que aminométhyle ou aminoéthyle,
- dialkylaminoalkyle tel que diméthylamino méthyle ou éthyle,
- dialkylaminoalkyloxy tel que diméthylamino éthyloxy,
- hydroxyle,
- carboxy libre, estérifié tel que alkoxy carbonyle par exemple méthoxy carbonyle ou éthoxy carbonyle, ou salifié par exemple par un atome de sodium ou de potassium,
- alkyle ayant de 1 à 8 atomes de carbone tel que méthyle, éthyle propyle, isopropyle, butyle, isobutyle, tert-butyle, éventuellement substitué par un ou plusieurs atome d'halogène, par exemple le fluor tel que le trifluorométhyle,
- oxo, cyano, nitro, formyl,
- acyle tel que acétyle, propionyle, butyryle, benzoyle,
- acyloxy tel que acétoxy ou un radical de formule :

   -O-CO-(CH₂)ₙCO₂H dans lequel n = 1 à 5,
- alkoxy tel que méthoxy, éthoxy, propyloxy, isopropyloxy, butyloxy,
- alkylthio tel que méthylthio, éthylthio, propylthio, isopropylthio, butylthio,
- carbamoyle,
- alcényle tel que vinyle, propényle,
- alcynyle tel que éthynyle, propynyle et
- aryle tel que phényle, furyle, thiènyle.

Comme exemple de tels radicaux substitués, on peut citer par exemple un radical alkyle substitué par un ou plusieurs atomes d'halogène, par exemple le fluor, tel que le radical trifluorométhyle, trifluorobutyle, pentafluoropropyle, pentafluorobutyle, pentafluoropentyle, heptafluorobutyle ou nonafluorobutyle, ou par exemple le chlore tel que le 2-chloroéthyle.

On peut également citer par exemple un radical aryle substitué par un ou plusieurs atomes d'halogène, par exemple le chlore, tel que le 4-chlorophényle.

L'invention s'étend naturellement aux sels des composés de formule (I), comme par exemple aux sels formés, lorsque les composés de formule (I) comportent une fonction amino, avec les acides chlorhydrique, bromhydrique, nitrique, sulfurique, phosphorique, acétique, formique, propionique, benzoïque, maléique, fumarique, succinique, tartrique, citrique, oxalique,glyoxylique, aspartique, alcane sulfonique tels que les acides méthane ou éthane sulfoniques, arylsulfonique, tels que les acides benzène ou paratoluène sulfonique et arylcarboxylique, ou lorsque les composés de formule (I) comportent une fonction acide, avec les sels des métaux alcalins ou alcalinoterreux ou d'ammonium éventuellement substitué.

L'invention a notamment pour objet les produits de formule générale (I) telle que définie précédemment répondant à la formule générale (I') : 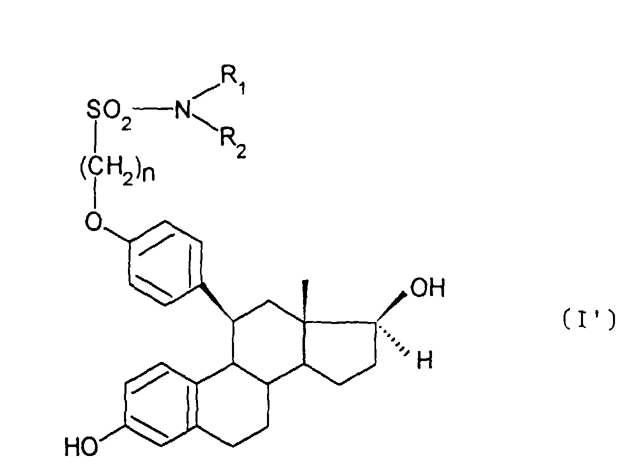 dans laquelle, R₁ et R₂ identiques ou différents représentent un atome d'hydrogène, un radical alkyle linéaire, ramifié ou cyclique renfermant de 1 à 8 atomes de carbone, éventuellement substitué, un radical acyle ayant au plus 12 atomes de carbone, ou un radical phényle éventuellement substitué,
- ou R₁ représente un radical carbamoyle monosubstitué par un radical alkyle linéaire, ramifié ou cyclique ayant au plus 8 atomes de carbone éventuellement substitué, ou par un radical phényle éventuellement substitué, et R₂ représente un atome d'hydrogène,
- ou R₁ et R₂ forment avec l'atome d'azote auquel ils sont liés, une urée cyclique du type 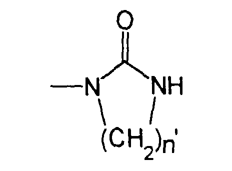 n' étant égal à 2 ou à 3,
- ou R₁ et R₂ forment un radical diméthylaminométhylène,
n est égal au plus à 7,
ainsi que les sels d'addition de ceux-ci.

L'invention a particulièrement pour objet les produits de formule générale (I) telle que définie précédemment dans laquelle R₁₇ est un radical hydroxyle et R'₁₇ représente un atome d'hydrogène.

L'invention a particulièrement pour objet les produits de formule générale (I) telle que définie précédemment dans laquelle n est égal à 5 ou 6.

L'invention a particulièrement pour objet les produits de formule générale (I) telle que définie précédemment dans laquelle R₁ et R₂ représentent un atome d'hydrogène.

L'invention a particulièrement pour objet les produits de formule générale (I) telle que définie précédemment dans laquelle R₁ et R₂ représentent un radical alkyle linéaire, ramifié ou cyclique ayant au plus 8 atomes de carbone éventuellement substitué par un ou plusieurs atomes d'halogènes.

L'invention a particulièrement pour objet les produits de formule générale (I) telle que définie précédemment dans laquelle R₁ représente un atome d'hydrogène et R₂ représente un radical alkyle linéaire, ramifié ou cyclique ayant au plus 8 atomes de carbone.

L'invention a particulièrement pour objet les produits de formule générale (I) telle que définie précédemment dans laquelle R₁ représente un radical carbamoyle monosubstitué par un radical alkyle, linéaire, ramifié ou cyclique renfermant de 1 à 8 atomes de carbone, ou par un radical phényle, chacun de ces radicaux étant éventuellement substitué par des atomes d'halogènes et R₂ représente un atome d'hydrogène.

L'invention a tout spécialement pour objet les produits, de formule générale (I) telle que définie précédemment, dont les noms suivent :
le N-butyl-5-[4-(3,17β-dihydroxy-estra-1,3,5(10)-trien-11β-yl)phénoxy]-pentanesulfonamide,
le N-butyl 5-[4-(3,17β-dihydroxy-estra-1,3,5(10)-trien-11β-yl)phénoxy)-N-méthyl-pentanesulfonamide,
le 5-[4-(3,17β-dihydroxy-estra-1,3,5(10)-trien-11β-yl)phénoxy]-N-(2,2,3,3,4,4,4-heptafluorobutyl)-N-méthyl-pentanesulfonamide.

L'invention a également pour objet un procédé de préparation des composés de formule (I), caractérisé en ce que l'on soumet un composé de formule (II) : 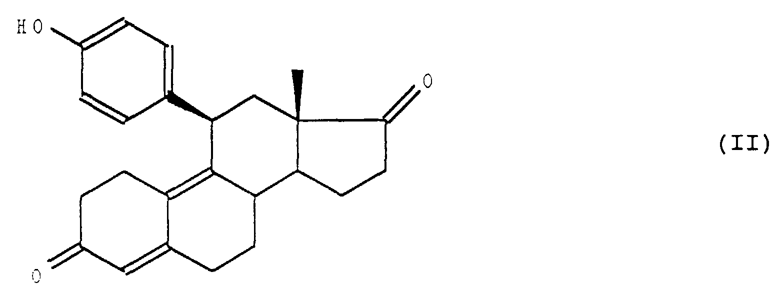 dans laquelle la fonction phénol est, le cas échéant, protégée,
**A) Soit** à l'action d'un dérivé halogéné de formule (III)

   X-(CH₂)ₙ-SO₂-NR_{A}R'_{A} (III)

   dans laquelle X est un atome d'halogène, n a la signification indiquée précédemment, R_{A} et R'_{A} identiques ou différents, représentent un atome d'hydrogène, un radical alkyle linéaire, ramifié ou cyclique renferment de 1 à 8 atomes de carbone éventuellement substitué, un radical acyle ayant au plus 12 atomes de carbone, éventuellement substitué, un radical aryle ou aralkyle éventuellement substitué tels que définis précedemment pour la formule (I), étant entendu que l'un au moins des substituants R_{A} et R'_{A} n'est pas un atome d'hydrogène, ou forment avec l'azote auquel ils sont liés un hétérocycle azoté saturé à 5 ou 6 chaînons renfermant éventuellement un second hétéroatome choisi parmi les atomes d'azote, d'oxygène et de soufre et éventuellement substitué par un groupement alkyle ayant de 1 à 4 atomes de carbone, pour obtenir un produit de formule (IV) : 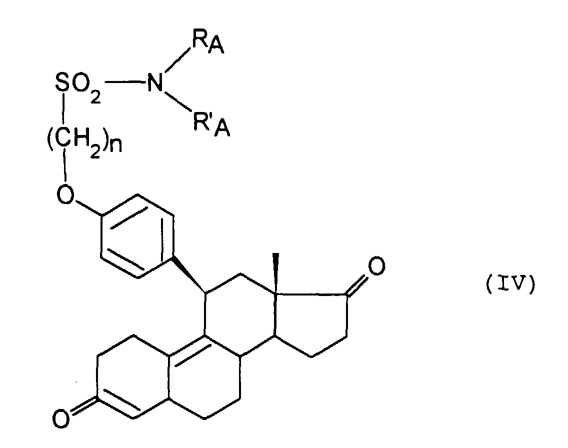 dans laquelle n, R_{A} et R'_{A} ont la même signification que précédemment, que l'on soumet à l'action d'un agent d'aromatisation du cycle A, et éventuellement d'acylation du radical hydroxyle en position 3, pour obtenir un produit de formule (I_{A}), correspondant au produit de formule (I), dans laquelle R₁=R_{A} et R₂=R'_{A}, 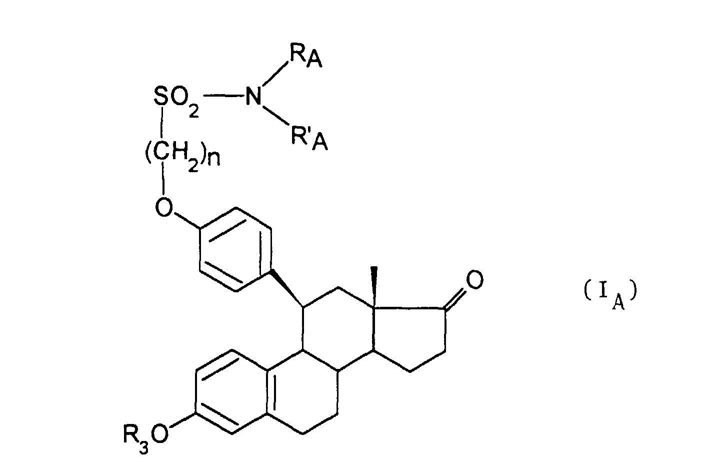 et dans laquelle n, R_{A} et R'_{A} ont la même signification que précédemment, et R₃ représente un atome d'hydrogène ou un groupement acyle ayant au plus 12 atomes de carbone, produit de formule (I_{A}) que l'on soumet, si désiré et si nécessaire, à l'une ou plusieurs des réactions suivantes, dans un ordre approprié:
   - réduction de la fonction cétone en 17
   - addition sur la fonction cétone en 17 d'un complexe métallique de formule (X) :

      M-R'₁₇ₐ (X)

      dans laquelle M représente un atome métallique et R'₁₇ₐ a la même signification que R'₁₇ à l'exception d'un atome d'hydrogène,
   - acylation sélective en position 17 lorsque R₁₇ est un hydroxyle,
   - alkylation ou acylation du radical hydroxyle en position 3,
   - saponification lorsque R₃ représente une fonction acyle,
   - salification éventuelle par un acide ou une base,
**B) Soit** à l'action d'un agent d'aromatisation du cycle A et à une réaction de protection du radical hydroxyle en position 3, puis à une réaction sélective d'élimination du groupement protecteur du phénol en position 11, pour obtenir un produit de formule (V) : 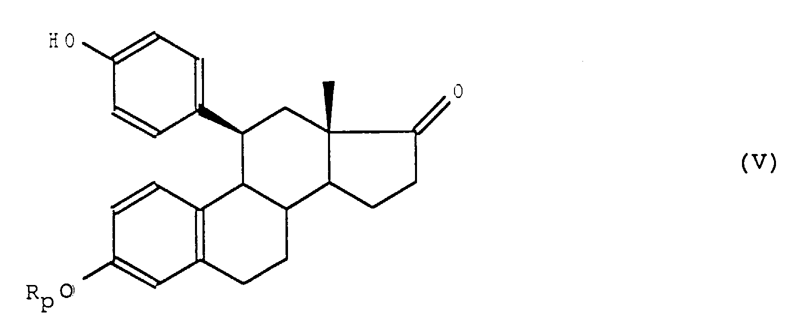 dans laquelle Rₚ représente un groupement protecteur, que l'on soumet à l'action d'un composé de formule (III') :

   X-(CH₂)ₙ-SO₂-N=CH-N(Alk₁)(Alk₂) (III')

   X étant un atome d'halogène, (Alk₁) et (Alk₂) étant des groupements alkyles ayant de 1 à 4 atomes de carbone et n est égal au plus à 18, puis à une réaction d'hydrolyse de l'imine formée, pour obtenir un composé de formule (VI) : 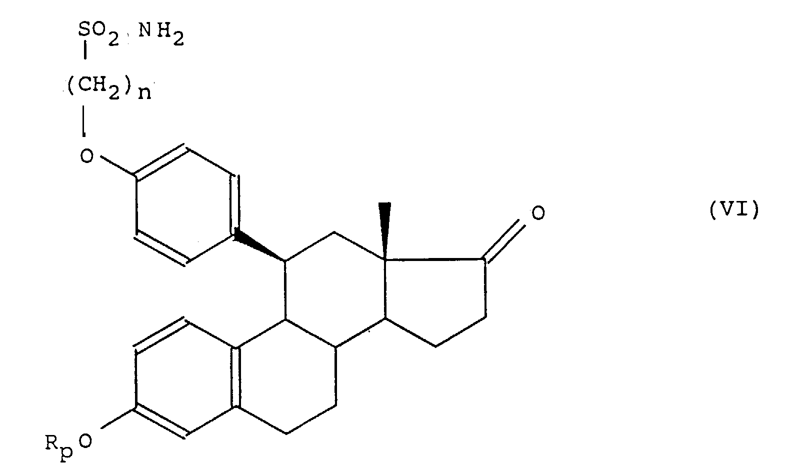 et dans laquelle n et Rₚ ont la même signification que précédemment, pouvant le cas échéant, selon la nature du groupement Rp correspondre à un produit de formule (I),
produit de formule (VI) que l'on soumet, si désiré et si nécessaire, à l'une ou plusieurs des réactions suivantes, dans un ordre approprié :
- réaction d'élimination du groupement protecteur Rₚ
- réduction de la fonction cétone en 17
- addition sur la fonction cétone en 17 d'un complexe métallique de formule (X) :

   M-R'₁₇ₐ (X)

   telle que définie précédemment,
- acylation sélective en position 17 lorsque R₁₇ est un hydroxyle.
- alkylation ou acylation du radical hydroxyle en position 3
- action d'un halogénure de formule (VII)

   R_{B}-X (VII)

   dans laquelle R_{B} est un radical alkyle ayant de 1 à 8 atomes de carbone éventuellement substitué, ou un radical acyle ayant au plus 12 atomes de carbone et X représente un atome d' halogène afin d'obtenir le composé de formule (I_{B}) correspondant au produit de formule (I) dans laquelle R₁= H et R₂=R_{B}, 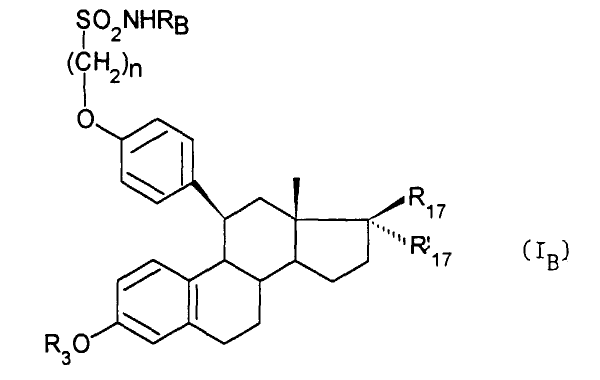 et dans laquelle n, R₁₇, R'₁₇ et R₃ ont les significations indiquées précédemment,
- action d'un isocyanate de formule (VIII) :

   R_{c}-NCO (VIII)

   dans laquelle R_{C} représente un radical alkyle linéaire, ramifié ou cyclique ayant au plus 8 atomes de carbone aryle, ou aralkyle tels que définis précedemment pour la formule (I) chacun des radicaux étant éventuellement substitués afin d'obtenir le composé de formule (I_{C}), correspondant au produit de formule (I) dans laquelle R₁ représente un radical carbamoyle monosubstitué et R₂ un atome d'hydrogène, 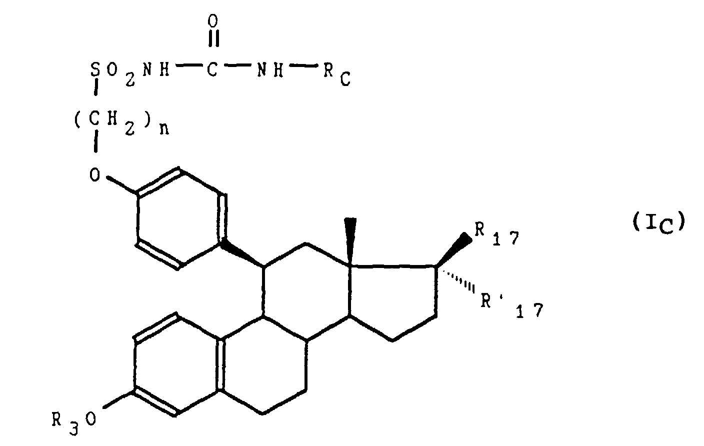 et dans laquelle n, R₁₇, R'₁₇ et R₃ ont les significations indiquées précédemment,
- action d'un composé de formule (IX) :

   (Alk₃O)(Alk₄O)CH-N(Alk₁)(Alk₂) (IX)

   dans laquelle Alk₁, Alk₂, Alk₃ et Alk₄ sont des radicaux alkyles ayant de 1 à 4 atomes de carbone, afin d'obtenir le composé de formule (I_{D}), correspondant au produit de formule (I) dans laquelle R₁ et R₂ forment un groupement dialkylaminométhylène, 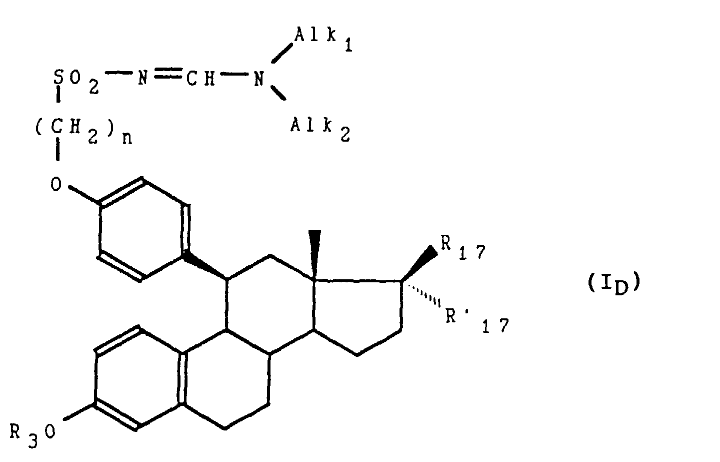 et dans laquelle n, R₁₇, R'₁₇ et R₃ ont les significations indiquées précédemment,
- alkylation ou acylation du produit de formule (I_{B}), par action d'un halogénure de formule (VII), afin d'obtenir un sulfonamide dialkylé, diacylé ou alkylacylé correspondant,
- réaction de cyclisation du produit de formule (I_{C}), lorsque R_{c} est un radical -(CH₂)ₙ,-Hal, n' étant égal à 2 ou 3 et Hal représente un atome d'halogène, afin d'obtenir le produit de formule (I'_{C}) correspondant au produit de formule (I) dans laquelle R₁ et R₂ forment ensemble avec l'azote auquel il sont liés une urée cyclique du type 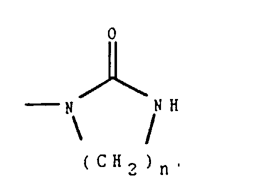
- salification éventuelle par un acide ou une base.

Les composés de formule (I_{A}), correspondant aux composés de formule (I) dans laquelle R₁=R_{A} et R₂=R'_{A}, sont obtenus en faisant réagir un composé de formule (II) avec successivement
a) un composé de formule (III) en présence d'une base forte telle que l'hydrure de sodium dans un solvant dipolaire aprotique tel que le diméthylformamide, en opérant, par exemple à température ambiante, afin d'obtenir le composé intermédiaire de formule (IV)
b) un agent d'aromatisation tel que le mélange bromure d'acétyle-anhydride acétique, suivie d'une réaction de saponification ménagée en présence par exemple de potasse dans le méthanol ou de soude dans le méthanol.

Les composés de formule (VI) sont obtenus en faisant réagir un composé de formule (II) dans laquelle le phénol en position 11 est éventuellement protégé avec successivement
a) un agent d'aromatisation tel que l'hydroxyde de palladium sur magnésie au sein du méthanol, suivie d'une réaction de protection de la fonction hydroxy en position 3 par action par exemple du chlorure de benzyle en présence de carbonate de potassium dans l'acétone, en opérant au reflux pendant 7 heures, et suivie d'une réaction de déprotection sélective de la fonction phénol en position 11, par exemple, par une saponification ménagée en présence par exemple de potasse dans le méthanol ou de soude dans le méthanol afin d'obtenir le composé intermédiaire de formule (V),
b) un composé de formule (III') en présence d'une base forte telle que l'hydrure de sodium dans un solvant dipolaire aprotique tel que le diméthylformamide,
c) un agent d'hydrolyse acide de l'imine formée au stade précédent, tel que par exemple de l'acide chlorhydrique dans le mélange méthanol-tetrahydrofuranne.

Les composés de formule (I_{B}), correspondant aux composés de formule (I) dans laquelle R₁= H et R₂ = R_{B}, sont obtenus en faisant réagir un composé de formule (VI), dans lequel ORₚ est un hydroxyle protégé, par exemple, par un radical aralkyle, avec un composé de formule (VII), la réaction étant effectuée par exemple en présence de soude dans l'acétone au reflux ou en présence d'hydrure de sodium dans le diméthylformamide à température ambiante.

Une seconde alkylation ou acylation peut être effectuée dans les mêmes conditions opératoires.

Les composés de formule (I_{c}), correspondant aux composés de formule (I) dans laquelle R₁= H et R₂ = CONH-R_{C}, sont obtenus en faisant réagir un composé de formule (VI), dans lequel le groupement protecteur de la fonction hydroxyle en position 3 est éliminé et la fonction cétone en position 17 est réduite, avec un composé de formule (VIII) en opérant en présence d'une base forte telle que l'hydrure de sodium dans un solvant dipolaire aprotique tel que le diméthylformamide, en opérant, par exemple à température ambiante.

Lorsque R_{C} est du type (CH₂)ₙ,-Hal, n' étant égal à 2 ou 3, il se produit in situ une réaction de cyclisation, le milieu étant très basique, pour former les composés de formule I'_{C}.

Les composés de formule (I_{D}) correspondant aux produits de formule (I) dans laquelle R₁ et R₂ forment un groupement dialkylaminométhylène, sont obtenus en faisant réagir un composé de formule (VI), dans lequel le groupement protecteur de la fonction hydroxyle en position 3 est éliminé et la fonction cétone en position 17 est réduite, avec un composé de formule (IX), en opérant par exemple dans le diméthylformamide à température ambiante.

Lorsque le composé de formule (I) comporte une fonction cétone en 17, on obtient :
- le composé 17 béta hydroxylé correspondant, par exemple par action d'un agent de réduction tel que le borohydrure de sodium dans un solvant neutre tel que le méthanol,
- le composé correspondant comportant un radical R'₁₇ représentant un radical alkyle, alcényle ou alcynyle, éventuellement substitué, par addition d'un composé (X) tel que par exemple un complexe du lithium, selon le procédé décrit dans le brevet européen EP 57115,

Il est bien entendu, que si R'₁₇ représente un radical alkyle, alcényle ou alcynyle, éventuellement substitué par une fonction réactive, celle-ci peut-être provisoirement protégée par les méthodes usuelles.

Lorsque le composé de formule (I) possède un groupe hydroxylé en position 3, on obtient le stéroïde alkylé correspondant par action d'un réactif d'alkylation tel qu'un iodure d'alkyle ou un sulfate d'alkyle par exemple le sulfate de méthyle ou le stéroïde acylé correspondant par action d'un réactif d'acylation usuel tel qu'un halogénure d'acyle par exemple le chlorure d'acétyle.

Lorsque le composé de formule (I) possède une fonction hydroxyle en position 17béta, on obtient le stéroïde 17béta acyloxylé correspondant par action d'un agent d'acylation sélective par exemple l'anhydride acétique dans la pyridine en présence éventuellement de 4-diméthylaminopyridine, ou tout autre moyen connu de l'homme du métier.

Les groupements protecteurs que l'on peut utiliser pour protéger les fonctions réactives, telles que par exemple la fonction hydroxyle, sont choisis parmi les groupements usuels de la chimie organique et plus particulièrement de la chimie des peptides. On trouvera une liste non exhaustive de ces groupements ainsi que les méthodes d'élimination correspondantes, dans le brevet français FR 2 499 995, ainsi que dans l'ouvrage suivant : Protective group in organic synthesis (GREENE et WUTS (1991) Ed. WILEY).

On peut citer par exemple, les radicaux acétyle, benzoyle ou terbutyldiméthylsilyle pour la protection du phénol en position 11 du composé de formule (II).

On peut citer par exemple, les radicaux acétyle, benzoyle ou benzyle pour la protection de l'hydroxyle en position 3 des composés de formules (V) et (VI).

Par exemple, la réaction de protection de l'hydroxyle en position 3 avec un groupement benzyle peut s'effectuer par action du chlorure de benzyle en présence d'une base forte telle que l'hydrure de sodium dans un solvant aprotique dipolaire tel que le diméthylformamide à température ambiante, ou bien par action du bromure de benzyle en présence d'une base moins forte telle que le carbonate de potassium dans l'acétone au reflux. Cette protection présente l'intérêt de résister aux réactions d'hydrolyse ou de saponification.

Lorsque les composés intermédiaires comportent des fonctions réactives protégées, on obtient le composé déprotégé correspondant par action d'agents usuels. On trouvera une liste non exhaustive de ces groupements ainsi que les méthodes d'élimination correspondantes, dans le brevet français FR 2 499 995, ainsi que dans l'ouvrage suivant : Protective group in organic synthesis (GREENE et WUTS (1991) Ed. WILEY).

A titre purement indicatif, lorsque le phénol est protégé par un groupement acétyle, la réaction d'élimination de ce groupement protecteur pourra s'effectuer à l'aide d'un agent de saponification tel que la potasse en milieu alcoolique,
lorsque le phénol est protégé par un groupement terbutyldiméthylsilyle, la réaction d'élimination de ce groupement protecteur pourra s'effectuer à l'aide d'un agent d'hydrolyse tel que l'acide chlorhydrique,
lorsque le groupement hydroxy en position 3 est protégé par un groupement benzyle, la réaction d'élimination de ce groupement protecteur pourra s'effectuer de préférence par hydrogénolyse par action, par exemple, d'hydrogène en présence de catalyseur palladium sur charbon actif dans le mélange acétate d'éthyle/éthanol/acide acétique.

Dans un mode de réalisation préféré de l'invention :
- on utilise les composés de formule (III) dans laquelle X est de préférence un atome d'iode,
- on utilise les composés de formule (III') dans laquelle X est de préférence un atome d'iode,
- on utilise les composés de formule (VII) dans laquelle X est de préférence un atome d'iode dans le cas où R_{B} est un radical alkyle, et un atome de chlore dans le cas où R_{B} est un radical acyle.

L'invention s'étend également à un procédé tel que défini plus haut, caractérisé en ce que l'on utilise au départ un composé correspondant à un composé de formule (II) comportant en position 17 les substituants R₁₇ et R'₁₇ tels que définis plus haut.

Il va de soi que dans la mise en oeuvre d'un tel procédé et ainsi qu'il a déjà été dit pour le radical R'₁₇, des protections intermédiaires des radicaux R₁₇ et R'₁₇ peuvent être nécessaires. Ces protections sont définies plus haut. Les produits de départ ci-dessus sont connus, par exemple, par la demande européenne publiée n° 0384482, ou préparables par les procédés décrits dans la présente demande, au départ des produits 17-oxo.

Les composés de formule (I) présentent d'intéressantes propriétés pharmacologiques.

L'étude des produits sur les récepteurs hormonaux a permis de mettre en évidence que :
- les composés de formule (I) possèdent des activités glucocorticoïdes ou antiglucocorticoïdes, progestomimétiques ou antiprogestomimétiques, androgènes ou anti-androgènes, anti-minéralocorticoïdes, oestrogènes ou anti-oestrogènes.

Les composés de formule (I) possèdent en particulier une remarquable activité anti-oestrogène et des propriétés anti-prolifératives comme le montrent les résultats des tests donnés plus loin.

Ces propriétés rendent les composés de formule (I) utilisables pour lutter contre les effets secondaires des glucocorticoïdes : ils permettent de lutter également contre les troubles dus à une hypersécrétion de glucocorticoïdes et notamment contre le vieillisement en général et plus particulièrement contre l'hypertension, le retardement de la cicatrisation, l'athérosclérose, l'ostéoporose, le diabète, l'obésité ainsi que la dépression de l'immunité et l'insomnie.

Ces produits peuvent également présenter un intérêt dans le traitement de certaines tumeurs, qui expriment les récepteurs hormonaux, pour lesquels les produits de formule (I) présentent une affinité.

Les composés de formule (I) qui possèdent les propriétés anti-progestomimétiques peuvent être utilisés pour préparer des contraceptifs originaux, comme agents d'interruption de grossesse ou comme agent d'induction du travail.

Ces produits peuvent ainsi être utilisés comme inducteurs de règles chez la femme et plus généralement chez les animaux femelles à sang chaud.

Les produits sont alors administrés pendant les périodes où la progestérone joue un rôle physiologique essentiel, c'est-à-dire notamment pendant la phase lutéale du cycle, au moment de la nidation (ou implantation de l'embryon) et pendant la grossesse. Une méthode de contraception selon l'invention consiste à administrer à la femme un au moins des produits de formule (I) pendant 1 à 5 jours se situant préférentiellement à la fin du cycle. Ce produit est administré alors préférentiellement par la voie orale ou in vagino mais il peut également être utilisé par la voie parentérale. Les produits peuvent également être utilisés par la voie endonasale.

Les produits de formule (I) possédant des propriétés anti-progestomimétiques peuvent également être utilisés contres les dérèglements hormonaux et, par ailleurs, ils peuvent présenter un intérêt dans le traitement des tumeurs hormono-dépendantes.

Leurs actions sur les sécrétions hypophysaires rendent les produits utilisables dans la ménopause.

Ces produits peuvent également être utilisés dans la synchronisation de l'oestrus et la synchronisation de la mise bas chez les animaux d'élevage, en particulier les bovins et les ovins.

Les produits peuvent également être utilisés pour contrôler la fertilité des animaux familiers tels que chiens ou chats.

Les composés de formule (I) peuvent également présenter des propriétés progestomimétiques et peuvent ainsi être utilisés dans le traitement des aménorrhées, des dysménorrhées et des insuffisances lutéales.

Les composés de formule (I) qui possèdent des propriétés anti-androgènes peuvent être utilisés dans le traitement des hypertrophies et du cancer de la prostate, de l'hyperandrogénie, de l'anémie, de l'hirsutisme et de l'acné. Ils peuvent également être utilisés pour la contraception chez l'homme.

Les composés de formule (I) qui possèdent des propriétés oestrogènes les rendent utilisables également dans le traitement des troubles liés à une hypofolliculinie, par exemple les aménorrhées, les dysménorrhées, les avortements répétés, les troubles prémenstruels ainsi que le traitement de la ménopause et de l'ostéoporose.

Les propriétés anti-oestrogènes et anti-prolifératives des composés de formule (I) les rendent utilisables dans le traitement des carcinomes hormono-dépendants, comme par exemple les carcinomes mammaires et ses métastases et dans le traitement des tumeurs bénignes du sein.

L'invention a donc pour objet les composés de formules (I) ainsi que les sels d'addition pharmaceutiquement acceptables, à titre de médicaments.

Parmi les médicaments de l'invention on peut citer particulièrement les composés décrits dans la partie expérimentale et tout particulièrement les produits des exemples 2, 7 et 8.

La posologie utile varie en fonction de l'affection à traiter et de la voie d'administration : elle peut varier par exemple de 1 mg à 100 mg par jour chez l'adulte par voie orale.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les composés de formule (IV) et (VI) sont des produits intermédiaires nouveaux et l'invention a donc également pour objet les composés de formule (IV) et (VI) à titre de produits industriels nouveaux et notamment à titre d'intermédiaires pour la mise en oeuvre du procédé de l'invention.

Parmi les produits intermédiaires nouveaux de l'invention, on peut citer ceux dont la préparation est donnée plus loin dans la partie expérimentale :
le N-butyl 5-[4-(3,17-dioxo-estra-4,9-dièn-11β-yl)phenoxy]-N-méthyl-pentanesulfonamide,
le 5-[4-(3,17-dioxo-estra-4,9-dièn-11β-yl)phénoxy]-N-(2,2,3,3,4,4,4-heptafluorobutyl)-N-méthyl-pentanesulfonamide,
le 5-[4-[17-oxo-3-[(phénylméthyl)oxy]-estra-1,3,5(10)-trien-11β-yl]phénoxy]-pentanesulfonamide.

Le composé de formule (II) nécessaire à la mise en oeuvre du procédé est décrit notamment dans la demande de brevet européen EP 0 384 842 (préparation de l'exemple 43).

Des exemples de préparation des produits de formules (III) et (III') figurent ci-après dans la partie expérimentale. Ces composés sont en général connus, et sont préparés par des procédés analogues à ceux décrits dans la partie expérimentale.

Les exemples suivants illustrent l'invention sans toutefois la limiter.

### Préparation 1 : N-[(diméthylamino)méthylène]-5-iodo-pentanesulfonamide

### STADE A : 5-chloropentane sulfonamide

A une solution, sous gaz inerte, de 2,5 g de chlorure de 5-chloropentane sulfonyle (préparation décrite dans : Bull. Soc. Chim. Belg. (1965) 74 21) dans 30 ml de tetrahydrofuranne, on ajoute goutte à goutte à 0/+5°C, 2,5 ml d'une solution aqueuse à 28 % d'ammoniaque. On laisse la température évoluer de 3°C à 16°C, et agite à température ambiante pendant 2 heures. On évapore ensuite le tétrahydrofuranne sous pression réduite, reprend par de l'eau, extrait avec du chlorure de méthylène, lave à l'eau puis avec une solution aqueuse de chlorure de sodium, sèche et évapore sous pression réduite. On obtient 2,01 g du produit attendu. (F=62°C).
**Spectre I.R. :** Trichlorométhane (CHCl₃)

| | |
|---|---|
| -NH₂ | 3448 cm⁻¹ |
| | 3352 cm⁻¹ |
| -SO₂- | 1344 cm⁻¹ |
| | 1150 cm⁻¹ |
| -NH₂ | 1545 cm⁻¹ |

### STADE B : 5-chloro-N-[(diméthylamino)méthylène]-pentanesulfonamide

A une solution, sous gaz inerte, de 1,5 g du produit obtenu au stade précédent, dans 8 ml de diméthylformamide, on ajoute goutte à goutte à température ambiante 1,29 ml de N,N-diméthylformamide-diméthyl acétal. On agite la solution à température ambiante pendant 3 heures, puis on coule dans une solution aqueuse à 1 % d'hydrogéno sulfate de sodium, extrait avec de l'acétate d'éthyle, lave à l'eau puis avec une solution aqueuse saturée de chlorure de sodium, sèche et évapore à sec sous pression réduite. On obtient 1,809 g du produit attendu.
**Spectre I.R. :** (CHCl₃)

| | |
|---|---|
| -N=CH-N< | 1629 cm⁻¹ |
| -SO₂- | 1349 cm⁻¹ |
| | 1119 cm⁻¹ |

### STADE C : N-[(diméthylamino)méthylène]-5-iodo-pentanesulfonamide

A une solution de 2,09 g du produit obtenu au stade précédent, dans 31,5 ml de méthyle éthyle cétone (M.E.C.), on ajoute 2,98 g de iodure de sodium et on porte au reflux pendant 4 heures. Après refroidissement, on ajoute de l'eau, extrait avec de l'acétate d'éthyle, lave à l'eau puis avec une solution aqueuse saturée de chlorure de sodium, sèche et évapore à sec sous pression réduite. On obtient 2,69 g du produit attendu.
**Spectre I.R. :** (CHCl₃)

| | |
|---|---|
| -N=CH-N< | 1629 cm⁻¹ |

### Préparation 2 : N-butyl-5-iodo-N-méthyl-pentanesulfonamide

### STADE A : N-butyl-5-chloro-N-méthyl-pentanesulfonamide

A une solution, sous gaz inerte, de 410 mg de chlorure de 5-chloropentane sulfonyle, dans 10 ml de chlorure de méthylène, on ajoute 0,47 ml de N-butylméthylamine. La température rature évolue de 13°C à 26°C, puis on ajoute après refroidissement à 0/+5°C, 0,55 ml de triéthylamine, et agite à température ambiante pendant 2 heures. On coule ensuite dans une solution aqueuse d'acide chlorhydrique 1M, extrait avec du chlorure de méthylène, lave à l'eau puis avec une solution aqueuse saturée de chlorure de sodium, sèche et évapore à sec sous pression réduite. On obtient 486 mg du produit attendu.
**Spectre I.R. :** (CHCl₃)

| | |
|---|---|
| -SO₂-N< | 1334 cm⁻¹, 1142 cm⁻¹ |

### STADE B : N-butyl-5-iodo-N-méthyl-pentanesulfonamide

A une solution de 449 mg du produit obtenu au stade précédent dans 4,5 ml de méthyl éthyl cétone, on ajoute 526 mg de iodure de sodium et agite au reflux pendant 4 heures. Après refroidissement et évaporation sous pression réduite de la méthyl éthyl cétone, on ajoute de l'eau, extrait avec de l'acétate d'éthyle, lave à l'eau puis avec une solution aqueuse saturée de chlorure de sodium, sèche et évapore à sec sous pression réduite. On obtient 572 mg du produit attendu.
**Spectre I.R. :** (CHCl₃)

| | |
|---|---|
| -SO₂-N< | 1334 cm⁻¹, 1141 cm⁻¹ |

### Préparation 3 : N-(2,2,3,3,4,4,4-heptafluorobutyl)-5-iodo-N-méthyl-pentanesulfonamide

### STADE A : 5-chloro-N-(2,2,3,3,4,4,4-heptafluorobutyl)-N-méthyl-pentanesulfonamide

A une solution de 200 mg de chlorure de 5-chloropentane sulfonyle, dans 5 ml de chlorure de méthylène, on ajoute, 500 mg de chlorhydrate de N-(2,2,3,3,4,4,4-heptafluorobutyl)-N-méthylamine, obtenu selon le brevet européen 384842 exemple 75, puis, à 0/+5°C, 0,55 ml de triéthylamine, et agite à température ambiante pendant 2 heures. On ajoute de l'eau, extrait avec du chlorure de méthylène, lave à l'eau puis avec une solution aqueuse saturée de chlorure de sodium, sèche et évapore à sec sous pression réduite. On obtient 378 mg du produit attendu.
**Spectre I.R. :** (CHCl₃)

| | |
|---|---|
| -SO₂-N< | 1354 cm⁻¹, 1341 cm⁻¹, 1148 cm⁻¹ |
| Impureté C=O | 1730 cm⁻¹ |

### STADE B : N-(2,2,3,3,4,4,4-heptafluorobutyl)-5-iodo-N-méthylpentanesulfonamide

A une solution de 355 mg du produit obtenu au stade précédent, dans 3 ml de méthyl éthyl cétone, on ajoute 300 mg de iodure de sodium et on porte au reflux pendant 4 heures. Après évaporation du solvant, on reprend le résidu avec de l'eau et on extrait avec de l'acétate d'éthyle. On lave avec une solution aqueuse de thiosulfate de sodium puis de chlorure de sodium, sèche et évapore à sec sous pression réduite. On obtient 425 mg du produit attendu sous la forme d'une huile incolore qui cristallise lentement.
**Spectre I.R. :** (CHCl₃)

| | |
|---|---|
| N-SO₂- | 1354 cm⁻¹, 1341 cm⁻¹, 1148 cm⁻¹ |

### PREPARATION DE L'EXEMPLE 1 : 11β-(4-hydroxyphényl)-3-[(phénylméthyl)oxy]-estra-1,3,5(10)-trièn-17-one

### STADE A : 11β-[4 -(benzoyloxy)phényl]-estra-4,9-diène-3,17-dione.

A une solution, sous gaz inerte, de 8,758 g de 11β-(4-hydroxyphényl)-estra-4,9-diène-3,17-dione (préparation selon l'exemple 43 du brevet EP 384842) dans 92 ml d'acétone et 27 ml de soude aqueuse 1M on ajoute goutte à goutte à 0°/+5°C 3 ml de chlorure de benzoyle. En fin d'introduction du chlorure de benzoyle, on observe la précipitation du benzoate, on agite la suspension 10 minutes au bain de glace, puis 30 minutes à température ambiante, on coule dans une solution aqueuse d'acide chlorhydrique 0,1M, extrait avec de l'acétate d'éthyle, lave avec une solution aqueuse saturée de chlorure de sodium, sèche et évapore à sec sous pression réduite. On obtient 12,98 g du produit brut que l'on cristallise dans le mélange chlorure de méthylène/éther isopropylique. On obtient 9,93 g du produit recherché. F = 196°C.
**Spectre I.R. :** (CHCl₃)

| | |
|---|---|
| >C=O en 17 + cétone de Ar-O-CO-Ph : | 1736 cm⁻¹ (F) |
| Cétone conjuguée : | 1659 cm⁻¹ |
| | 1602 cm⁻¹ |
| C=C aromatiques : | 1505 cm⁻¹ |
| | 1490 cm⁻¹ |

### STADE B : 11β-[4-(benzoyloxy)phényl]-3-hydroxy-estra-1,3,5(10)-trièn-17-one.

A une solution de 10,07 g du produit obtenu comme au stade précédent, dans 104 ml de méthanol, on ajoute 10,1 g d'hydroxyde de palladium à 20 % sur oxyde de magnésium. On chauffe au reflux pendant 1 heure et 30 minutes. Après refroidissement, on filtre la suspension, lave le catalyseur insoluble avec un mélange chlorure de méthylène/méthanol 50/50 et évapore à sec sous pression réduite le filtrat. Le résidu (10,72 g) est chromatographié sur silice (éluant : acétate d'éthyle/cyclohexane 40/60). On obtient 7,72 g de produit recherché. F=265°C
**Spectre I.R. :** (CHCl₃)

| | |
|---|---|
| -OH : | 3599 cm⁻¹ |
| >C=O : | 1733 cm⁻¹ (F) |
| | 1611 cm⁻¹ |
| | 1602 cm⁻¹ |
| C=C aromatiques : | 1585 cm⁻¹ |
| | 1508 cm⁻¹ |

### STADE C : 11β-[4-(benzoyloxy)phényl]-3-[(phénylméthyl)oxy]estra-1,3,5(10)-trièn-17-one.

A une solution de 6,462 g du produit obtenu au stade précédent, dans 110 ml d'acétone, on ajoute 3,82 g de carbonate de potassium et 4,9 ml de bromure de benzyle (Fluka A007832). On chauffe au reflux pendant 7 heures puis après refroidissement, on évapore à sec sous pression réduite. On reprend le résidu avec de l'acétate d'éthyle et coule dans une solution aqueuse d'acide chlorhydrique 0,5M. On réextrait avec de l'acétate d'éthyle et on lave avec une solution aqueuse saturée de chlorure de sodium, sèche et évapore à sec sous pression réduite. Le résidu (12,2 g) est chromatographié sur silice (éluant : acétate d'éthyle/cyclohexane 25/75). On obtient 6,68 g de produit recherché.
**Spectre I.R. :** (CHCl₃)

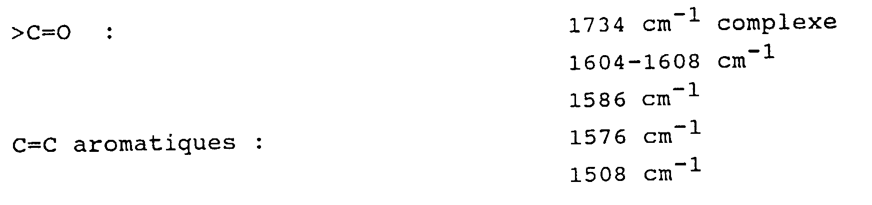

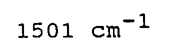

### STADE D : 11β-(4-hydroxyphényl)-3-[(phénylméthyl)oxy]-estra-1,3,5(10)-trièn-17-one.

A une solution de 6,64 g du produit obtenu au stade précédent, dans 87,5 ml de méthanol et 87,5 ml de tetrahydrofuranne, on ajoute 12 ml d'une solution aqueuse de soude 2M. On agite pendant 1 heure à température ambiante puis on coule dans une solution aqueuse d'acide chlorhydrique 0,5M. On extrait à l'acétate d'éthyle, lave à l'eau puis avec une solution aqueuse saturée de bicarbonate de sodium, puis avec une solution aqueuse saturée de chlorure de sodium, sèche et évapore à sec sous pression réduite. On obtient 6,52 g du produit brut attendu que l'on cristallise dans le chlorure de méthylène. On obtient 4,62 g du produit recherché. F = 240°C.
**Spectre I.R. :** (CHCl₃)

| | |
|---|---|
| -OH : | 3600 cm⁻¹ |
| >C=O : | 1733 cm⁻¹ |
| | 1613 cm⁻¹ |
| | 1594 cm⁻¹ |
| C=C aromatiques : | 1574 cm⁻¹ |
| | 1513 cm⁻¹ |
| | 1500 cm⁻¹ |

### EXEMPLE 1 : 5-[4-(3,17β-dihydroxy-estra-1,3,5(10)-trien-11β-yl)phénoxy]-pentanesulfonamide.

### STADE A : N-[(diméthylamino)méthylène]-5-[4-[17-oxo-3-[(phénylméthyl)oxy]-estra-1,3,5(10)-trien-11β-yl]phénoxy]-pentanesulfonamide.

A une suspension, sous gaz inerte, de 2,92 g du produit obtenu au stade D de la préparation de l'exemple 1, dans 69 ml de diméthylformamide et de 403 mg d'hydrure de sodium à 50 % dans l'huile, après agitation 25 minutes, on ajoute une solution de 2,67 g du produit obtenu dans la préparation 1 (stade C), dans 25 ml de diméthylformamide. On chauffe la suspension à 50°C et obtient au bout de 10 minutes une solution que l'on maintient sous agitation à cette température pendant 1 heure et 15 minutes. On refroidit à température ambiante et coule dans une solution aqueuse à 1 % d'hydrogénosulfate de sodium, extrait à l'acétate d'éthyle, lave à l'eau puis avec une solution saturée de thiosulfate de sodium et avec une solution aqueuse saturée de chlorure de sodium, sèche et évapore à sec sous pression réduite. Le résidu (6,75 g) est chromatographié sur silice (éluant : acétate d'éthyle). On obtient 3,82 g de produit recherché.
**Spectre I.R. :** (CHCl₃)

| | |
|---|---|
| >C=O en position 17 : | 1733 cm⁻¹ |
| -N=CH- : | 1629 cm⁻¹ |
| | 1611 cm⁻¹ (épaulement) |
| | 1575 cm⁻¹ |
| C=C aromatiques : | 1512 cm⁻¹ |
| | 1500 cm⁻¹ |
| -SO₂- : | 1349 cm⁻¹ |

### STADE B : 5-[4-[17-oxo-3-[(phénylméthyl)oxy]-estra-1,3,5(10)-trien-11β-yl] phénoxy]-pentanesulfonamide.

A une suspension, sous gaz inerte, de 3,818 g du produit obtenu au stade précédent, dans 59 ml de methanol et de 28 ml de tétrahydrofuranne, on ajoute 17,7 ml d'acide chlorhydrique concentré pur 22°Be. On chauffe à 80°C pendant 1 heure et 30 minutes, puis, après avoir refroidi, on coule dans une solution aqueuse saturée de bicarbonate de sodium, on extrait à l'acétate d'éthyle et lave avec une solution aqueuse saturée de chlorure de sodium, sèche et évapore à sec sous pression réduite. Le résidu (4,15 g) est chromatographié sur silice (éluant : acétate d'éthyle/cyclohexane 60/40). On obtient 2,89 g de produit recherché.
**Spectre I.R. :** (CHCl₃)

| | |
|---|---|
| -NH₂ | 3444 cm⁻¹ |
| | 3350 cm⁻¹ |
| >C=O en position 17 : | 1733 cm⁻¹ |
| | 1610 cm⁻¹ |
| C=C aromatiques : | 1580 cm⁻¹ |
| + | |
| -NH₂ : | 1545 cm⁻¹ |

### STADE C : 5-[4-[17β-hydroxy-3-[(phénylméthyl)oxy]-estra-1,3,5(10)-trien-11β-yl]phénoxy]-pentanesulfonamide.

A une solution, sous gaz inerte, de 1,36 g du produit obtenu au stade précédent, dans 6 ml de méthanol et de 6 ml de tétrahydrofuranne, on ajoute à 0°/+5°C 169 mg d'hydrure de bore et de sodium. Après agitation à 0°/+5°C pendant 1 heure, on coule dans une solution aqueuse d'acide chlorydrique 1M, extrait avec de l'acétate d'éthyle, lave à l'eau puis avec une solution saturée de chlorure de sodium, sèche et évapore à sec sous pression réduite. On obtient 1,299 g du produit brut attendu que l'on cristallise dans le mélange chlorure de méthylène/éther isopropylique. On obtient 1,295 g du produit recherché. F = 146°C
**Spectre I.R**. **:** (CHCl₃)

| | |
|---|---|
| -OH : | 3609 cm⁻¹ |
| -NH₂ : | 3444 cm⁻¹ |
| | 3353 cm⁻¹ |
| | 1609 cm⁻¹ |
| C=C aromatiques : | 1580 cm⁻¹ |
| | 1512 cm⁻¹ , 1500 cm⁻¹ |
| -NH₂ : | 1544 cm⁻¹ |

### STADE D : 5-[4-(3,17β-dihydroxy-estra-1,3,5(10)-trien-11β-yl)phénoxy]-pentanesulfonamide.

A une solution de 1,295 g du produit obtenu au stade précédent, dans 50 ml d'éthanol et 5 ml d'acide acétique, on ajoute 161 mg de palladium 10 % sur charbon actif (type E 10N, Degussa), puis on agite sous une pression de 1640 mbar d'hydrogène pendant 2 heures. On filtre, lave avec un mélange méthanol/chlorure de méthylène 1/1, évapore à sec sous pression réduite et on chasse l'acide acétique par entraînement avec du toluène. On obtient 1,04 g du produit brut attendu que l'on cristallise dans l'éthanol. On obtient 736 mg du produit recherché. F = 175°C
**Spectre I.R. :**
(Nujol)Absorption complexe région OH/NH

| | |
|---|---|
| | 1616 cm⁻¹ |
| C=C aromatiques : | 1580 cm⁻¹ |
| + -NH₂ | 1511 cm⁻¹ |
| -SO₂ | 1333 cm⁻¹ |
| | 1153 cm⁻¹ |

### EXEMPLE 2 : N-butyl-5-[4-(3,17β-dihydroxy-estra-1,3,5(10)-trien-11β-yl)phénoxy]-pentanesulfonamide.

### STADE A : N-butyl-5-[4-[17-oxo-3-[(phénylméthyl)oxy]-estra-1,3,5(10)-trien-11β-yl]phénoxy]-pentanesulfonamide.

A une solution, sous gaz inerte, de 500 mg du produit obtenu au stade B de l'exemple 1, dans 6,5 ml d'acétone et 0,96 ml d'une solution aqueuse de soude 1M, on ajoute 0,188 ml de 1-iodobutane. On agite au reflux pendant 52 heures puis on refroidit avant d'évaporer sous pression réduite l'acétone. On reprend ensuite avec de l'acétate d'éthyle et coule dans une solution aqueuse d'acide chlorhydrique 0,5M, extrait avec de l'acétate d'éthyle, lave avec de l'eau puis successivement avec une solution saturée de thiosulfate de sodium et une solution aqueuse saturée de chlorure de sodium, sèche et évapore à sec sous pression réduite. On recueille 612 mg de produit que l'on chromatographie sur silice (éluant acétate d'éthyle/cyclohexane 50/50). On recueille ainsi 68 mg du produit recherché. (Rf = 0,40 Acétate d'éthyle/Cyclohexane 50/50)
**Spectre I.R. :** (CHCl₃)

| | |
|---|---|
| -NH- : | ∼3400 cm⁻¹ |
| >C=O : | 1733 cm⁻¹ |
| | 1610 cm⁻¹ |
| C=C aromatiques : | 1579 cm⁻¹ |
| | 1511 cm⁻¹ |
| | 1510 cm⁻¹ |
| -SO₂- | 1327 cm⁻¹ + 1141 cm⁻¹ |

### STADE B : N-butyl-5-[4-[17β-hydroxy-3-[(phénylméthyl)oxy]-estra-1,3,5(10)-trien-11β-yl]phénoxy]-pentanesulfonamide.

A une solution sous gaz inerte, refroidie à 0/+5°C comprenant 294 mg du produit obtenu au stade précédent, dans 1,3 ml de tétrahydrofuranne et 1,3 ml de méthanol, on ajoute 33 mg d'hydrure de bore et de sodium. On agite 1 heure à 0/+5°C, coule dans une solution aqueuse d'acide chlorhydrique 1M, extrait avec de l'acétate d'éthyle, lave avec une soluton aqueuse saturée de chlorure de sodium, sèche et évapore à sec sous pression réduite. On recueille 300 mg de produit que l'on chromatographie sur silice (éluant acétate d'éthyle / cyclohexane 50/50). On recueille ainsi 194 mg du produit recherché. (Rf = 0,25 Acétate d'éthyle/Cyclohexane 50/50).

### STADE C : N-butyl-5-[4-(3,17β-dihydroxy-estra-1,3,5(10)trien-11β-yl)phénoxy]-pentanesulfonamide.

A une solution comprenant 194 mg du produit obtenu au stade précédent, dans 5 ml d'acétate d'éthyle, 5 ml d'éthanol et 5 ml d'acide acétique, on ajoute 44 mg du catalyseur palladium à 10 % sur charbon actif, puis on agite sous une pression de 1700 mbar d'hydrogène pendant 15 minutes. On filtre la suspension, lave avec un mélange méthanol/chlorure de méthylène 1/1, évapore le filtrat à sec sous pression réduite, entraîne l'acide acétique avec du toluène et sèche sous pression réduite. On obtient 172 mg de produit que l'on chromatographie sur silice (éluant chlorure de méthylène/isopropanol 96/4). On recueille ainsi 142 mg du produit recherché.
**Spectre I.R. :** (CHCl₃)

| | |
|---|---|
| -OH : | 3601 cm⁻¹ |
| -NH- : | 3400 cm⁻¹ |
| | 1610 cm⁻¹ |
| C=C aromatiques : | 1581 cm⁻¹ |
| | 1512 cm⁻¹ |
| -SO₂- | 1327 cm⁻¹ |
| | 1140 cm⁻¹ |

### EXEMPLE 3 : 5-[4-(3,17β-dihydroxy-estra-1,3,5(10)-trien-11β-yl)phénoxy]-N-[(diméthylamino)méthylène]-pentanesulfonamide.

A une solution de 200 mg du produit obtenu au stade D de l'exemple 1, dans 1,5 ml de diméthylformamide, on ajoute 0,062 ml de N,N-diméthylformamide-diméthylacétal et agite pendant 1 heure et 30 minutes à température ambiante. On coule dans une solution aqueuse à 1 % d'hydrogénosulfate de sodium, extrait avec de l'acétate d'éthyle, lave à l'eau, puis avec une solution aqueuse saturée de chlorure de sodium, sèche et évapore à sec sous pression réduite. On recueille 227 mg de produit que l'on chromatographie sur silice (éluant chlorure de méthylène/isopropanol 95/5). On recueille ainsi 168 mg du produit recherché.
**Spectre I.R. :** (CHCl₃)

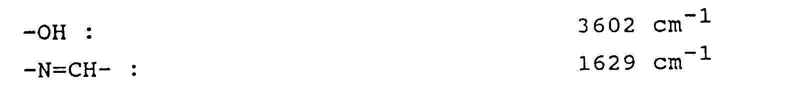

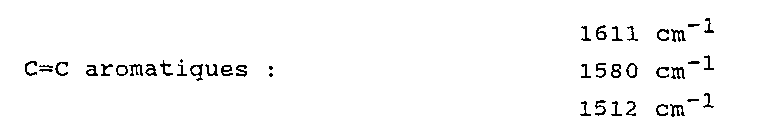

### EXEMPLE 4 : N-butyl-N'-[5-[4-(3,17β-dihydroxy-estra-1,3,5(10)-trien-11β-yl)phénoxy]pentylsulfonyl]-urée

A une solution, sous gaz inerte, de 200 mg du produit obtenu au stade D de l'exemple 1, dans 2 ml de diméthylformamide, on ajoute 20 mg d'hydrure de sodium et, après 10 minutes d'agitation à température ambiante, 0,046 ml d'isocyanate de butyle. Après 1 heure d'agitation à température ambiante, on coule dans une solution aqueuse d'acide chlorhydrique 1M, extrait avec de l'acétate d'éthyle, lave avec une solution aqueuse saturée de chlorure de sodium sèche et évapore à sec sous pression réduite. On recueille 317 mg de produit que l'on chromatographie sur silice (éluant chlorure de méthylène/isopropanol 95/5). On recueille ainsi 146 mg du produit recherché.
**Spectre I.R. :** (nujol)

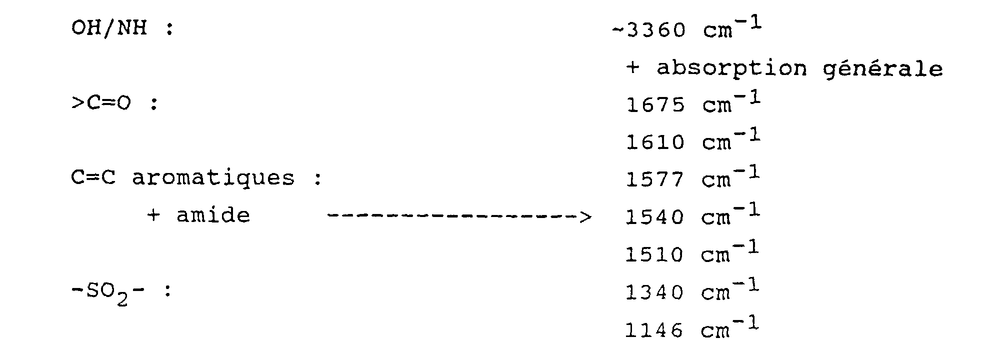

### EXEMPLE 5 : N-(4-chlorophényl)-N'-[5-[4-(3,17β-dihydroxy-estra-1,3,5(10)-trien-11β-yl)phénoxy]pentylsulfonyl]-urée

A une solution, sous gaz inerte, de 183 mg du produit obtenu au stade D de l'exemple 1, dans 2 ml de diméthylformamide, on ajoute 22 mg d'hydrure de sodium à 50 % et, après 10 minutes d'agitation à température ambiante, 73 mg de 4-chloro-phényl isocyanate. Après 5 heures d'agitation à température ambiante, on coule dans une solution aqueuse d'acide chlorhydrique 1M, extrait avec de l'acétate d'éthyle, lave avec une solution aqueuse saturée de chlorure de sodium, sèche et évapore à sec sous pression réduite. On obtient 289 mg de produit que l'on chromatographie sur silice (éluant chlorure de méthylène/isopropanol 92,5/7,5). On recueille ainsi 86 mg du produit recherché.
**Spectre I.R. :** (nujol)
Absorption complexe OH/NH :

| | |
|---|---|
| >C=O : | ∼1698 cm⁻¹ |
| | 1607 cm⁻¹ |
| C=C aromatiques: | 1540 cm⁻¹ |
| + amide | 1511 cm⁻¹ |
| | 1494 cm⁻¹ |

### EXEMPLE 6 : 1-[5-[4-(3,17β-dihydroxy-estra-1,3,5(10)-trien-11β-yl)phénoxy]-pentylsulfonyl]-2-imidazolidinone

A une solution, sous gaz inerte, de 200 mg du produit obtenu au stade D de l'exemple 1, dans 2 ml de diméthylformamide, on ajoute 20 mg d'hydrure de sodium à 50 % et, après 10 minutes d'agitation à température ambiante, 0,035 ml de 2-chloroéthyl isocyanate. Après 1 heure et 30 minutes d'agitation à température ambiante, on coule dans une solution aqueuse d'acide chlorhydrique 1M, extrait avec de l'acétate d'éthyle, lave avec de l'eau puis avec une solution aqueuse saturée de chlorure de sodium, sèche et évapore à sec sous pression réduite. On obtient 278 mg de produit que l'on chromatographie sur silice (éluant acétate d'éthyle puis chlorure de méthylène/isopropanol 92,5/7,5). On recueille ainsi 102 mg du produit recherché.
**Spectre I.R. :** (nujol)
Absorption générale région OH/NH :

| | |
|---|---|
| >C=O : | 1726 cm⁻¹ |
| | 1612 cm⁻¹ |
| C=C aromatiques : | 1580 cm⁻¹ |
| | 1510 cm⁻¹ |
| | 1505 cm⁻¹ (épaulement) |
| -SO₂- : | 1155 cm⁻¹ |

### EXEMPLE 7 : N-butyl-5-[4-(3,17β-dihydroxy-estra-1,3,5(10)-trien-11β-yl)phénoxy]-N-méthyl-pentanesulfonamide.

### STADE A : N-butyl-5-[4-(3,17-dioxo-estra-4,9-dièn-11β-yl)phénoxy]-N-méthyl-pentanesulfonamide.

A une suspension, sous gaz inerte, de 58 mg d'hydrure de sodium à 50 % dans l'huile dans 6 ml de diméthylformamide, on ajoute 362,5 mg de 11β-(4-hydroxyphényl)-estra-4,9-diène-3,17-dione (préparation de l'exemple 43 du brevet EP 384842), après 30 minutes d'agitation à température ambiante, on ajoute une solution de 417 mg du produit obtenu dans la préparation 2 (stade B), dans 1,5 ml de diméthylformamide. On laisse la température évoluer de 23°C à 27°C au cours de l'introduction puis on agite pendant 45 minutes. On coule dans une solution aqueuse d'acide chlorhydrique 1M, extrait avec de l'acétate d'éthyle, lave à l'eau puis successivement avec une solution saturée de thiosulfate de sodium et une solution aqueuse saturée de chlorure de sodium, sèche et évapore à sec sous pression réduite. Le résidu (883 mg) est chromatographié sur silice (éluant : acétate d'éthyle/ cyclohexane 60/40). On obtient 433 mg du produit recherché.
**Spectre I.R. :** (CHCl₃)

| | |
|---|---|
| >C=O en position 17 : | 1735 cm⁻¹ |
| diènone | 1658 cm⁻¹ |
| | 1609 cm⁻¹ |
| C=C + | 1600 cm⁻¹ (épaulement) |
| C=C aromatiques : | 1580 cm⁻¹ |
| | 1509 cm⁻¹ (Fort) |
| | 1333 cm⁻¹ |
| -SO₂N< : | 1140 cm⁻¹ |

### STADE B : N-butyl-5-[4-(3,17β-dihydroxy-estra-1,3,5(10)-trien-11β-yl)phénoxy]-N-méthyl-pentanesulfonamide.

1) Aromatisation
A une solution, sous gaz inerte, de 404,5 mg du produit obtenu au stade précédent, dans 4 ml de chlorure de méthylène, on ajoute à 0/+5°C 0,32 ml d'anhydride acétique et 0,16 ml de bromure d'acétyle. On agite 15 minutes à cette température puis 1 heure 15 minutes à température ambiante.
2) Saponification de l'acétate
On ajoute au mélange réactionnel refroidi à 0/+5°C, 0,3 ml de méthanol et après avoir agité pendant 10 minutes, on évapore à sec sous pression réduite à température ambiante. On reprend avec 2,4 ml de méthanol et 2,4 ml de tétrahydrofuranne et on ajoute 0,47 ml de lessive de soude. On agite à température ambiante pendant 45 minutes.
3) Réduction de la cétone en 17
On ajoute au mélange réactionnel refroidi à 0/+5°C, 131 mg d'hydrure de bore et de sodium. Après agitation à température ambiante pendant 45 minutes, on coule dans une solution aqueuse d'acide chlorydrique 1M, puis on extrait avec de l'acétate d'éthyle, lave à l'eau, puis avec une solution saturée de chlorure de sodium, sèche et évapore à sec sous pression réduite. Le résidu (393 mg) est chromatographié sur silice (éluant : acétate d'éthyle/cyclohexane 50/50). On obtient 193 mg du produit recherché.
**Spectre I.R. :** (CHCl₃)

| | |
|---|---|
| -0H : | 3600 cm⁻¹ |
| | 1610 cm⁻¹ |
| C=C aromatiques : | 1581 cm⁻¹ |
| | 1512 cm⁻¹ |
| -SO₂N< : | 1332 cm⁻¹ |
| | 1138 cm⁻¹ |

### EXEMPLE 8 : 5-[4-(3,17β-dihydroxy-estra-1,3,5(10)-trien-11β-yl)phénoxy]-N-(2,2,3,3,4,4,4-heptafluorobutyl)-N-méthylpentanesulfonamide.

### STADE A : 5-[4-(3,17-dioxo-estra-4,9-dièn-11β-yl)phénoxy]-N-(2,2,3,3,4,4,4-heptafluorobutyl)-N-méthyl-pentanesulfonamide.

A une solution de 255 mg de 11β-(4-hydroxyphényl)-estra-4,9-diène-3,17-dione (préparation de l'exemple 43 du brevet EP 384842), dans 4,5 ml de diméthylformamide, on ajoute 40 ml d'hydrure de sodium à 50 % dans l'huile. Après 30 minutes d'agitation à température ambiante, on ajoute 400 mg du produit obtenu à la préparation 3 (stade B) et on agite à température ambiante pendant 45 minutes. On coule dans une solution aqueuse d'acide chlorhydrique 1M, extrait avec de l'acétate d'éthyle, lave successivement avec une solution saturée de thiosulfate de sodium et une solution aqueuse saturée de chlorure de sodium, sèche et évapore à sec sous pression réduite. Le résidu (600 mg) est chromatographié sur silice (éluant : acétate d'éthyle/Essence G 55/45). On obtient 335 mg du produit recherché.
**Spectre I.R. :** (CHCl₃)

| | |
|---|---|
| >C=O en position 17 : | 1735 cm⁻¹ |
| diènone | 1658 cm⁻¹ |
| | 1609 cm⁻¹ |
| C=C + | 1580 cm⁻¹ |
| C=C aromatiques : | 1509 cm⁻¹ |
| | 1342 cm⁻¹ |
| -SO₂N< : | 1148 cm⁻¹ |

### STADE B : 5-[4-(3,17β-dihydroxy-estra-1,3,5(10)-trien-11β-yl)phénoxy]-N-(2,2,3,3,4,4,4-heptafluorobutyl)-N-méthylpentanesulfonamide

1) Aromatisation
A une solution de 430 mg du produit obtenu au stade précédent, dans 3,5 ml de chlorure de méthylène, on ajoute à 0°/+5°C 0,35 ml d'anhydride acétique et 0,20 ml de bromure d'acétyle et agite 40 minutes à température ambiante.
2) Saponification de l'acétate
On ajoute au mélange réactionnel refroidi à 0°/+5°C, 0,5 ml de méthanol et on évapore à sec sous pression réduite. On reprend avec 3,5 ml de méthanol et on ajoute 0,6 ml de lessive de soude.
3) Réduction de la cétone en 17
On ajoute au mélange réactionnel, 228 mg d'hydrure de bore et de sodium. Après agitation à température ambiante pendant 20 minutes, on acidifie à pH=2 avec de l'acide chlorydrique 2N, puis on extrait avec du chlorure de méthylène, sèche et évapore à sec sous pression réduite. Le résidu (429 mg) est chromatographie sur silice (éluant : acétate d'éthyle/essence G 40/60). On obtient 266 mg du produit recherché. (F=136°C-138°C)
**Spectre I.R. :** (CHCl₃)

| | |
|---|---|
| -0H : | 3615 cm⁻¹ |
| | 1610 cm⁻¹ |
| C=C aromatiques : | 1581 cm⁻¹ |
| | 1512 cm⁻¹ |
| | 1491 cm⁻¹ |
| -SO₂N< : | 1342 cm⁻¹ |
| | 1148 cm⁻¹ |

### Compositions pharmaceutiques :

On a préparé des comprimés répondant à la formule suivante :
- Produit de l'exemple **7** 50 mg
- Excipient (talc, amidon, stéarate de magnésium) Q.S. pour un comprimé terminé à 120 mg

On a préparé des suspensions injectables répondant à la formule suivante :
- Produit de l'exemple **7** 25 mg
- Excipient (solution aqueuse dispersive) : alcool benzylique, polysorbate 80, carboxyméthylcellulose (sel de sodium), chlorure de sodium, eau pour préparation injectable pour flacon de 1 ml

### Etude pharmacologique des produits de l'invention

### 1 - Etude de l'activité des produits de l'invention sur les récepteurs hormonaux.

On utilise soit le récepteur hormonal naturel de rat (AR), soit le récepteur humain recombinant (PR, GR et ER).

### Récepteur androgène de la prostate de rat :

Des rats mâles Sprague Dawley EOPS de 180 à 200 g sont castrés. 24 heures après la castration, les animaux sont sacrifiés, les prostates sont prélevées, pesées et homogénéisées à 0°C à l'aide d'un Potter teflon-verre dans une solution tamponnée TS (Tris 10 mM, saccharose 0,25 M, DTT 2mM, MoNa 20mM, PMSF 0,1mM, pH 7,4) (1 g de tissu pour 8 ml de TS). L'homogénat est ensuite centrifugé (209 000 g pendant 30 minutes) à 0°C. Des aliquotes du surnageant ainsi obtenu, sont incubées à 0°C pendant un temps d'incubation de 24 h avec une concentration constante (T) de testostérone tritiée en présence de concentrations croissantes, soit de testostérone froide (0 à 1000 x 10⁻⁹M), soit du produit à tester (1 à 25000 x 10⁻⁹M. La concentration de testostérone tritiée liée (B) est ensuite mesurée dans chaque incubat par la technique d'adsorption au charbon-dextran.

### Récepteur progestérone humain :

Le récepteur progestérone humain recombinant est obtenu par surexpression dans un système cellules d'insectes-Baculovirus, selon la méthodologie générale décrite par N.R. WEBB et al. (Journal of Methods in Cell and Molecular Biology, (1990) Vol 2 n° 4, 173-188) et dont l'application est décrite à l'expression des récepteurs hormonaux humains, par exemple le récepteur glucocorticoïde humain (G. SRINIVASAN et al. Molecular Endocrinology (1990) vol 4 n° 2 209-216).

On utilise le kit BaculoGold Transfection Kit (PharMingen, référence 21000K) pour insérer le fragment d'ADNc décrit par P. KASTNER et al. (The EMBO Journal (1990) vol 9 n° 5, 1603-1614), comprenant la région codante pour le récepteur progestérone humain et pour préparer le virus recombinant correspondant.

Le virus recombinant ainsi obtenu est utilisé pour exprimer le récepteur progestérone dans les cellules d'insectes SF9 (ATCC CRL1711), selon la méthodologie connue citée précédemment.

2 x 10⁷ à 2,5 x 10⁷ cellules SF9 sont cultivées dans un flacon "Falcon" de 175 cm² dans le milieu TNM-FH "SIGMA" supplémenté avec 10 % de sérum de veau foetal (SVF) et avec 50 microgramme/ml de gentamycine. Après infection puis incubation à 27°C pendant 40 à 42 heures, les cellules sont lysées dans 1 ml de tampon de lyse (1) par un cycle de congélation-décongélation que l'on répète encore deux fois. Le surnageant, contenant le récepteur progestérone humain recombinant est conservé dans l'azote liquide par dose de 1 ml.

Le surnageant est dilué au moment de l'emploi selon une gamme de dilution variant de 1/10^{e} au 1/100^{e} avec du tampon Tris 10mM, saccharose 0,25 M, HCl à pH 7,4 contenant 0,1 % de gélatine, puis incubés à 0°C pendant 24 heures avec une concentration constante (T) de 17α,21-diméthyl 19-nor-pregna 4,9-dièn-3,20-dione tritié en présence de concentrations croissantes soit de progestérone froide (0-2500 x 10⁻⁹M), soit du produit froid à tester (1 à 25000 x 10⁻⁹M). La concentration de 17,21-diméthyl 19-nor-4,9-prégnane-3,20-dione tritié lié (B) est ensuite mesurée dans chaque incubat par la technique d'adsorption au charbon dextran.

### Récepteur glucocorticoïde humain :

Un surnageant de cellules SF9 contenant le récepteur glucocorticoïde humain recombinant est obtenu selon le procédé décrit ci-dessus pour le récepteur progestérone en utilisant le fragment d'ADNc décrit par S.M. HOLLENBERG et al. (Nature (1985) vol. 318 n° 19/26 635) comprenant la région codante pour le récepteur glucocorticoïde humain. Les cellules obtenues sont lysées dans le tampon de lyse (2).

Le surnageant est incubé à 0°C pendant 24 heures avec une concentration constante (T) de 11β,17β-dihydroxy-6,21-diméthyl pregna 1,4,6-trièn-20-yn-3-one tritié en présence de concentrations croissantes soit de dexaméthasone froide (0-1000 x 10⁻⁹M) , soit du produit froid à tester (1 à 25000 x 10⁻⁹M). La concentration de 11β,17β-dihydroxy-6,21-diméthyl pregna 1,4,6-trièn-20-yn-3-one tritié lié (B) est ensuite mesurée dans chaque incubat par la technique d'adsorption au charbon dextran.

### Récepteur oestrogène humain :

Un surnageant de cellules SF9 contenant le récepteur oestrogène humain recombinant est obtenu selon le procédé décrit ci-dessus pour le récepteur progestérone en utilisant le fragment d'ADNc décrit dans le vecteur d'expression HEGO par L. TORA et al. (The EMBO Journal (1989) vol. 8 n° 7 1981-1986), comprenant la région codante pour le récepteur oestrogène humain de "type sauvage" avec une glycine en position 400. Les cellules obtenues sont lysées dans le tampon de lyse (1).

Le surnageant est incubé à 0°C pendant 24 heures avec une concentration constante (T) d'oestradiol tritié en présence de concentrations croissantes soit d'oestradiol froid (0-1000 x 10⁻⁹M), soit du produit froid à tester (1 à 25000 x 10⁻⁹M). La concentration d'oestradiol tritié lié (B) est ensuite mesurée dans chaque incubat par la technique d'adsorption au charbon dextran.

### Expression des résultats et méthodes de calcul

- Calcul de l'affinité relative de liaison (ARL).

On trace les 2 courbes suivantes : pourcentage de l'hormone tritiée liée B/BO en fonction du logarithme de la concentration de l'hormone de référence froide ou en fonction du logarithme de la concentration du produit froid testé.

On détermine la droite d'équation suivante :
I₅₀ = 100 (BO/BO+Bmin/BO)/2 doit I₅₀ = 100 (1+Bmin/BO)/2 = 50 (1+Bmin/BO)
BO = Concentration de l'hormone tritiée liée en l'absence de tout produit froid,
B = Concentration de l'hormone tritiée liée en présence d'une concentration X de produit froid,
B min = Concentration de l'hormone tritiée liée en présence d'un grand excès de l'hormone froide de référence (500 nM).

Les intersections de la droit I₅₀ et des courbes, permettent d'évaluer les concentrations de l'hormone de référence froide (CH) et du produit froid testé (CX) qui inhibent de 50 % la liaison spécifique de l'hormone tritiée sur le récepteur.

L'affinité relative de liaison (ARL) du produit testé est déterminé par l'équation :

ARL = 100 (CH)/(CX)

Les ARL des produits de référence Estradiol, Progestérone, Déxaméthasone et Testostérone sont prises arbitrairement égales à 100.
Les résultats des ARL obtenus sont les suivants :

| Produits des exemples | hER Estradiol = 100 | hGR Dexaméthasone = 100 | hPR Progestérone = 100 | AR Testostérone = 100 |
|---|---|---|---|---|
| 2 | 21 | 82 | 37 | 3 |
| 7 | 4 | 152 | 111 | 2,5 |
| 8 | 19 | 114 | 55 | 1,5 |

### Conclusion :

Les produits étudiés, en particulier les produits des exemples 2 et 8 ont une affinité marquée pour le récepteur oestrogène.

Les produits des exemples 2,7 et 8 ont une affinité marquée pour les récepteurs glucocorticoïde et progestérone.
Tampons de lyse : (1) Tris-HCl pH 8 : 20mM, EDTA : 0,5mM, DTT : 2mM, glycérol : 20 %, KCl : 400mM, PIC 1‰. (2) Phosphate de potassium pH 7,0 : 50mM, DTT : 5mM, glycérol : 20 %, Molybdate de sodium : 20mM, PIC 1‰. PIC : leupeptine, pepsatine A, Aprotinine, Antipaïne, Chymostatine. Concentration finale de chacun : 2,5 µg/ml
2 - Activité anti-proliférative des produits de l'invention sur la croissance de cellules tumorales mammaires humaines MCF-7

### Description du test :

a) Culture cellulaire :
   Les lignées MCF-7 sont maintenues en culture en milieu de base (d'après 1) contenant 5 % de sérum de veau foetal, à 37°C en atmosphère humide contenant 5 % CO₂. Les cellules à subconfluence sont récoltées par trypsination (trypsine 0,1 %, EDTA 0,02 %) puis rincées par centrifugation douce. Un échantillon des cellules en suspension est compté sur cellule de Malassez.
b) Etude de la croissance :
   Les cellules sont resuspendues dans un milieu de base sans rouge de phénol, en présence de 5 % de SVF déstéroïdé, et stimulées soit par 0,1 nM d'estradiol, soit par 10 ng/ml d'EGF + 1 ng/ml de PDGF. Les cellules sont ensuite ensemencées à raison de 50 000 cellules par puits dans des plaques multipuits (24 puits de 2,5 cm²). Vingt quatre heures après l'ensemencement (JO), le produit à tester est ajouté au milieu en solution éthanolique (concentration finale en éthanol : 0,1 %), à la concentration de 10⁻¹¹ à 10⁻⁶M, les puits contrôlés recevant la même concentration en éthanol. Les milieux contenant les produits sont renouvelés toutes les 48 heures. En fin d'expérience (J7 à J9), le milieu est aspiré et les cellules sont immédiatement fixées par 250 microlitres de méthanol afin de doser l'ADN.
   L'activité anti-proliférative des produits est évaluée par leur capacité à inhiber l'augmentation d'ADN relativement au contrôle.
c) Dosage de l'ADN :
   L'ADN est dosé par une méthode fluorimétrique utilisant le DABA (Acide 3,5 diaminobenzoïque) (d'après 2) : 200 micro-litres de DABA sont ajoutés dans chaque puits ; les plaques sont alors incubées 45 mn à 56°C, puis 2 ml d'HCL 1N sont ajoutés. La fluorescence est mesurée à l'aide d'un fluorimètre (longueur d'onde excitatrice : 408 nm, longueur d'onde d'émission : 510 nm).

La quantité d'ADN par puits est évaluée par rapport à une gamme étalon obtenue en traitant dans les mêmes conditions un standard d'ADN de thymus de veau.

### Résultats :

La concentration en nM qui inhibe de 50 % la croissance des cellules MCF-7 stimulée par EGF + PDGF (CI₅₀) a été déterminée de la manière indiquée ci-dessus.

### Résultats :

- Produit de l'exemple 2 :: CI₅₀ = 0,016 nM
- Produit de l'exemple 7 :: CI₅₀ = 0,015 nM
- Produit de l'exemple 8 :: CI₅₀ = 0,026 nM

(1) Un milieu de base est préparé comme suit : Milieu MEM (Minimal Essential Medium) auquel sont ajoutés :
   - acides aminés non essentiels (GIBCO) 1 %,
   - péni-strepto (pénicilline 100 U/ml, streptomycine 0,1 mg/ml),
   - fungizone 0,1 %,
   - glutamine 2 mM,
   - bicarbonate de sodium 2,25 mg/ml.
(2) Puzas et Goodman, Analytical Biochemistry, Vol 86, p. 50, 1978.

## Revendications

1. Les composés de formule (I) : 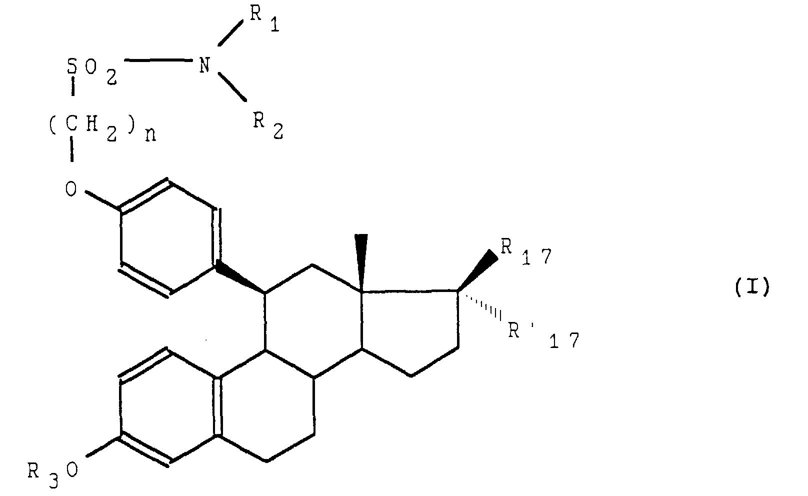 dans laquelle R₁₇ et R'₁₇ sont tels que :
- soit R₁₇ et R'₁₇ forment ensemble une cétone
- soit R₁₇ représente un radical hydroxyle, ou un radical acyloxy ayant au plus 12 atomes de carbone et R'₁₇ représente un atome d'hydrogène, un radical alkyle, alcényle ou alcynyle ayant au plus 8 atomes de carbone, chacun de ces radicaux étant éventuellement substitué,
- R₃ représente un atome d'hydrogène, un radical alkyle linéaire, ramifié ou cyclique ayant au plus 8 atomes de carbone ou un radical acyle ayant au plus 12 atomes de carbone,
et dans laquelle, R₁ et R₂ identiques ou différents représentent un atome d'hydrogène, un radical alkyle linéaire, ramifié ou cyclique renfermant de 1 à 8 atomes de carbone, éventuellement substitué, un radical acyle ayant au plus 12 atomes de carbone, un radical aryle ou aralkyle, chacun de ces radicaux étant éventuellement substitué, dans lesquels le radical alkyle renferme au plus 6 atomes de carbone et le radical aryle représente un radical mono ou polycyclique pouvant renfermer un ou plusieurs hétéroatomes choisis parmi les atomes d'oxygène, d'azote et de soufre,
ou R₁ représente un radical carbamoyle monosubstitué par un radical alkyle linéaire, ramifié ou cyclique ayant au plus 8 atomes de carbone, éventuellement substitué, ou par un radical aryle ou aralkyle, chacun de ces radicaux étant éventuellement substitué, dans lesquels le radical alkyle renferme au plus 6 atomes de carbone et le radical aryle représente un radical mono ou polycyclique pouvant renfermer un ou plusieurs hétéroatomes choisis parmi les atomes d'oxygène, d'azote et de soufre et R₂ représente un atome d'hydrogène,
- ou R₁ et R₂ forment avec l'atome d'azote auquel ils sont liés un hétérocycle azoté saturé à 5 ou 6 chainons renfermant éventuellement un second hétéroatome, choisi parmi les atomes d'azote, d'oxygène et de soufre, éventuellement substitué par un radical alkyle ayant de 1 à 4 atomes de carbone, ou par un groupe oxo,
- ou R₁ et R₂ forment un radical dialkylaminométhylène, chaque alkyle renfermant de 1 à 4 atomes de carbone,
n est un entier égal au plus à 18,
ainsi que les sels d'addition de ceux-ci.

2. Les composés de formule générale (I) telle que définie dans la revendication 1, répondant à la formule générale (I') : 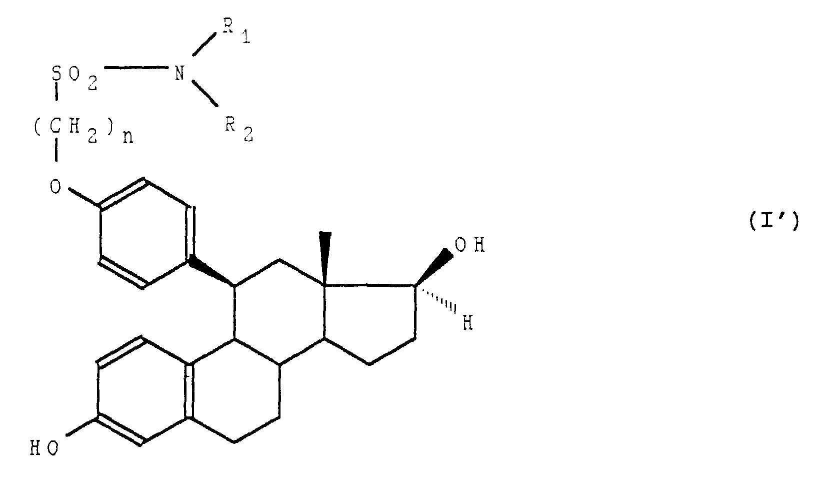 dans laquelle, R₁ et R₂ identiques ou différents, représentent :
un atome d'hydrogène, un radical alkyle linéaire, ramifié ou cyclique renfermant de 1 à 8 atomes de carbone, éventuellement substitué, un radical acyle ayant au plus 12 atomes de carbone, ou un radical phényle éventuellement substitué,
ou R₁ représente un radical carbamoyle monosubstitué par un radical alkyle linéaire, ramifié ou cyclique ayant au plus 8 atomes de carbone éventuellement substitué, ou par un radical phényle éventuellement substitué, et R₂ représente un atome d'hydrogène,
- ou R₁ et R₂ forment avec l'atome d'azote auquel ils sont liés, une urée cyclique du type 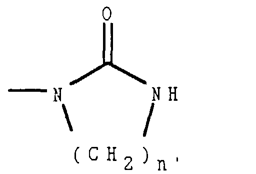 n' étant égal à 2 ou à 3,
- ou R₁ et R₂ forment un radical diméthylaminométhylène,
n est égal au plus à 7,
ainsi que les sels d'addition de ceux-ci.

3. Les composés de formule (I) telle que définie à la revendication 1 ou 2, dans laquelle R₁₇ est un radical hydroxyle et R'₁₇ représente un atome d'hydrogène.

4. Les composés de formule (I) telle que définie à la revendication 1,2 ou 3 dans laquelle n est égal à 5 ou 6

5. Les composés de formule (I) telle que définie à l'une quelconque des revendications 1 à 4, dans laquelle R₁ et R₂ représentent un atome d'hydrogène.

6. Les composés de formule (I) telle que définie à l'une quelconque des revendications 1 à 4, dans laquelle R₁ et R₂ représentent un radical alkyle linéaire, ramifié ou cyclique ayant au plus 8 atomes de carbone éventuellement substitués par 1 ou plusieurs atomes d'halogènes.

7. Les composés de formule (I) telle que définie à l'une quelconque des revendications 1 à 4, dans laquelle R₁ représente un atome d'hydrogène et R₂ représente un radical alkyle linéaire, ramifié ou cyclique ayant au plus 8 atomes de carbone.

8. Les composés de formule (I) telle que définie à l'une quelconque des revendications 1 à 4, dans laquelle R₁ représente un radical carbamoyle monosubstitué par un radical alkyle, linéaire, ramifié ou cyclique renfermant de 1 à 8 atomes de carbone, ou par un radical phényle, chacun de ces radicaux étant éventuellement substitué par des atomes d'halogènes et R₂ représente un atome d'hydrogène.

9. Les composés de formule (I) telle que définie à la revendication 1 dont les noms suivent :
le N-butyl-5-[4-(3,17β-dihydroxy-estra-1,3,5(10)-trien-11β-yl)phénoxy]-pentanesulfonamide,
le N-butyl-5-[4-(3,17β-dihydroxy-estra-1,3,5(10)-trien-11β-yl)phénoxy]-N-méthyl-pentanesulfonamide,
le 5-[4-(3,17β-dihydroxy-estra-1,3,5(10)-trien(11β-yl)phénoxy]-N-(2,2,3,3,4,4,4-heptafluorobutyl)-N-méthylpentanesulfonamide.

10. Procédé de préparation des composés de formule (I) telle que définie à la revendication 1, caractérisé en ce que l'on soumet un composé de formule (II) : 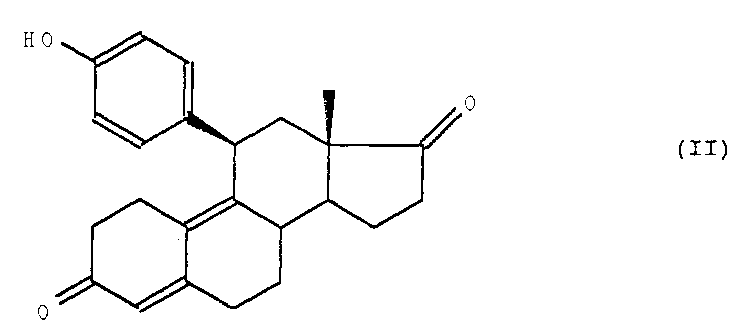 dans laquelle la fonction phénol est, le cas échéant, protégée,
**A) Soit** à l'action d'un dérivé halogéné de formule (III) :
X-(CH₂)ₙ-SO₂-NR_{A}R'_{A} (III)
dans laquelle X est un atome d'halogène, n a la signification indiquée dans la revendication 1, R_{A} et R'_{A} identiques ou différents, représentent un atome d'hydrogène, un radical alkyle linéaire, ramifié ou cyclique renfermant de 1 à 8 atomes de carbone éventuellement substitué, un radical acyle ayant au plus 12 atomes de carbone, un radical aryle ou aralkyle éventuellement substitué, tels que définis à la revendication 1, étant entendu que l'un au moins des substituants R_{A} et R'_{A} n'est pas un atome d'hydrogène, ou forment avec l'azote auquel ils sont liés un hétérocycle azoté saturé à 5 ou 6 chaînons renfermant éventuellement un second hétéroatome choisi parmi les atomes d'azote, d'oxygène et de soufre et éventuellement substitué par un groupement alkyle ayant de 1 à 4 atomes de carbone, pour obtenir un produit de formule (IV) : 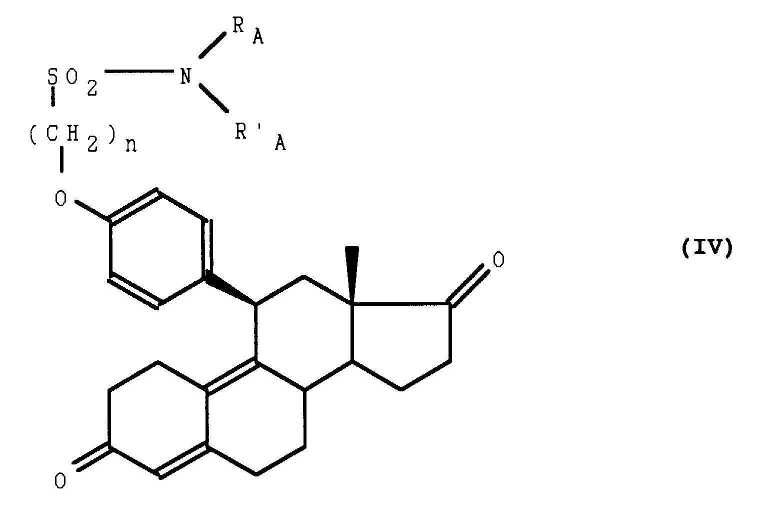 dans laquelle n, R_{A} et R'_{A} ont la même signification que précédemment, que l'on soumet à l'action d'un agent d'aromatisation du cycle A, et éventuellement d'acylation du radical hydroxyle en position 3, pour obtenir un produit de formule (I_{A}), correspondant au produit de formule (I), dans laquelle R₁=R_{A} et R₂=R'_{A}, 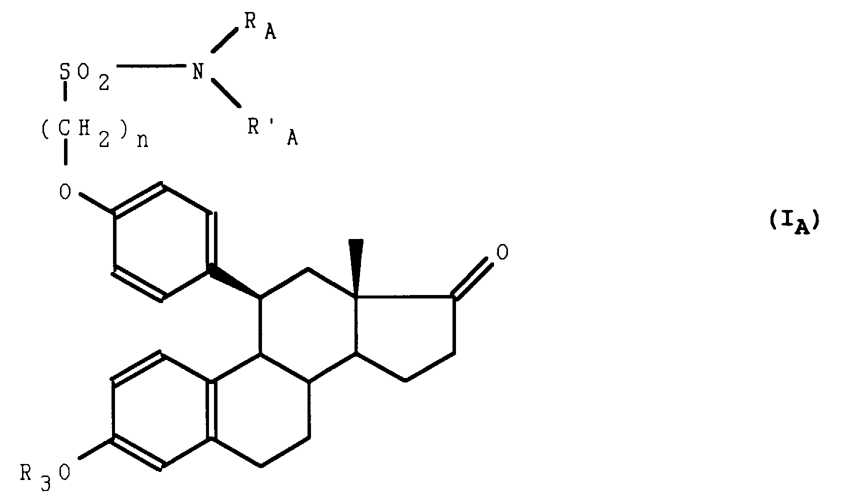 et dans laquelle n, R_{A} et R'_{A} ont la même signification que précédemment, et R₃ représente un atome d'hydrogène ou un groupement acyle ayant au plus 12 atomes de carbone, produit de formule (I_{A}) que l'on soumet, si désiré et si nécessaire, à l'une ou plusieurs des réactions suivantes, dans un ordre approprié:
- réduction de la fonction cétone en 17
- addition sur la fonction cétone en 17 d'un complexe métallique de formule (X) :
M-R'₁₇ₐ (X)
dans laquelle M représente un atome métallique et R'₁₇ₐ a la même signification que R'₁₇ à l'exception d'un atome d'hydrogène,
- acylation sélective en position 17 lorsque R₁₇ est un hydroxyle,
- alkylation ou acylation du radical hydroxyle en position 3
- saponification lorsque R₃ représente une fonction acyle
- salification éventuelle par un acide ou une base
**B) Soit** à l'action d'un agent d'aromatisation du cycle A et à une réaction de protection du radical hydroxyle en position 3, puis à une réaction sélective d'élimination du groupement protecteur du phénol en position 11, pour obtenir un produit de formule (V) : 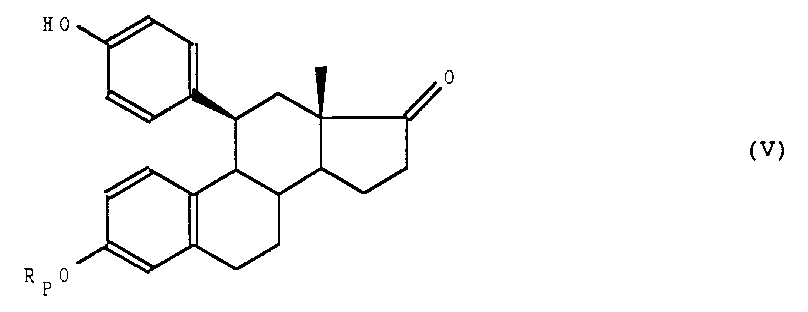 dans laquelle Rₚ représente un groupement protecteur, que l'on soumet à l'action d'un composé de formule (III'),
X-(CH₂)ₙ-SO₂-N=CH-N(Alk₁)(Alk₂) (III')
X étant un atome d'halogène, (Alk₁) et (Alk₂) étant des groupements alkyles ayant de 1 à 4 atomes de carbone et n est égal au plus à 18, puis à une réaction d'hydrolyse de l'imine formée, pour obtenir un composé de formule (VI) : 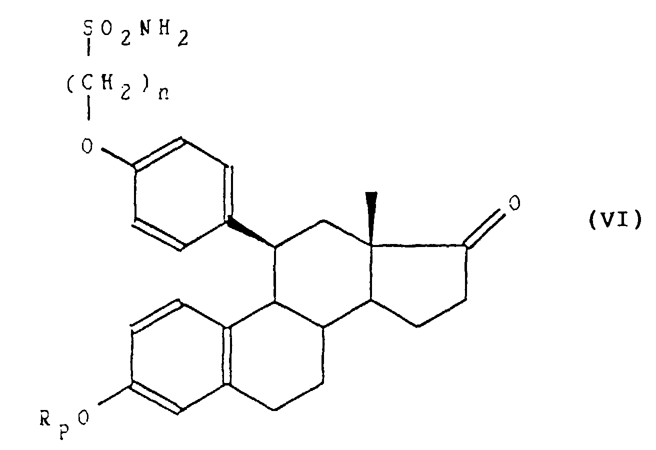 et dans laquelle n et Rₚ ont la même signification que précédemment, pouvant le cas échant, selon la nature du groupement Rₚ correspondre à un produit de formule (I), produit de formule (VI) que l'on soumet, si désiré et si nécessaire, à l'une ou plusieurs des réactions suivantes, dans un ordre approprié :
- réaction d'élimination du groupement protecteur Rₚ
- réduction de la fonction cétone en 17
- addition sur la fonction cétone en 17 d'un complexe métallique de formule (X) :
M-R'₁₇ₐ (X)
telle que définie précédemment,
- acylation sélective en position 17 lorsque R₁₇ est un hydroxyle,
- alkylation ou acylation du radical hydroxyle en position 3
- action d'un halogénure de formule (VII)
R_{B}-X (VII)
dans laquelle R_{B} est un radical alkyle ayant de 1 à 8 atomes de carbone éventuellement substitué, ou un radical acyle ayant au plus 12 atomes de carbone et X représente un atome d'halogène afin d'obtenir le composé de formule (I_{B}) correspondant au produit de formule (I) dans laquelle R₁=H et R₂=R_{B}, 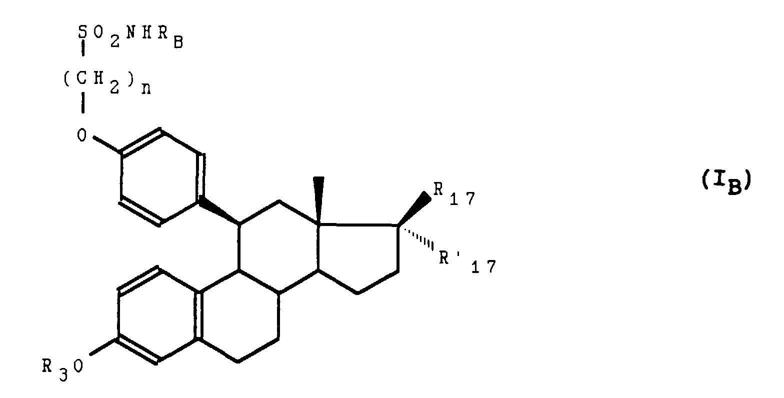 et dans laquelle n, R₁₇, R'₁₇ et R₃ ont les significations indiquées dans la revendication 1,
- action d'un isocyanate de formule (VIII)
R_{C}-NCO (VIII)
dans laquelle R_{C} représente un radical alkyle linéaire, ramifié ou cyclique ayant au plus 8 atomes de carbone, aryle ou aralkyle tels que définis à la revendication 1, chacun des radicaux étant éventuellement substitués afin d'obtenir le composé de formule (I_{C}), correspondant au produit de formule (I) dans laquelle R₁ représente un radical carbamoyle monosubstitué et R₂ un atome d'hydrogène, 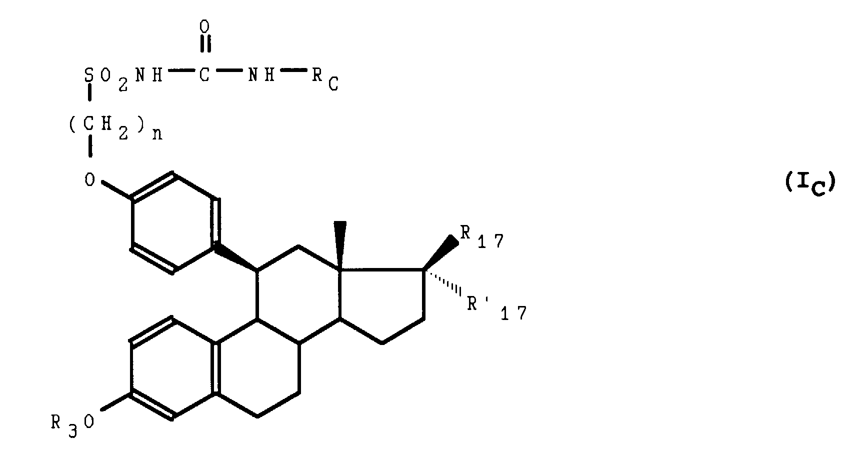 et dans laquelle n, R₁₇, R'₁₇ et R₃ ont les significations indiquées dans la revendication 1,
- action d'un composé de formule (IX)
(Alk₃O)(Alk₄O)CH-N(Alk₁)(Alk₂) (IX)
dans laquelle Alk₁, Alk₂, Alk₃ et Alk₄ sont des radicaux alkyles ayant de 1 à 4 atomes de carbone, afin d'obtenir le composé de formule (I_{D}), correspondant au produit de formule (I) dans laquelle R₁ et R₂ forment un groupement dialkylaminométhylène, 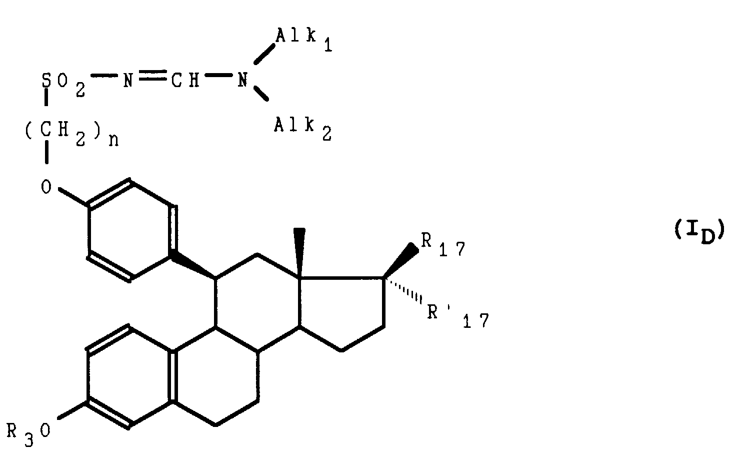 et dans laquelle n, R₁₇, R'₁₇ et R₃ ont les significations indiquées dans la revendication 1,
- alkylation ou acylation du produit de formule (I_{B}), par action d'un halogénure de formule (VII), afin d'obtenir un sulfonamide dialkylé, diacylé ou alkylacylé correspondant,
- réaction de cyclisation du produit de formule (I_{C}), lorsque R_{c} est un radical -(CH₂)_{n'}-Hal, n' étant égal à 2 ou 3 et Hal représente un atome d'halogène, afin d'obtenir le produit de formule (I'_{C}) correspondant au produit de formule (I) dans laquelle R₁ et R₂ forment ensemble avec l'azote auquel il sont liés une urée cyclique du type 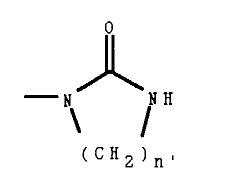
- salification éventuelle par un acide ou une base.

11. Procédé de préparation des produits de formule (I) telle que définie à la revendication 1, caractérisé en ce que l'on exécute le procédé de façon analogue à la revendication 10 et qu'on utilise au départ un composé correspondant au composé de formule (II) telle que définie à la revendication 10 comportant en position 17 les substituants R₁₇ et R'₁₇ tels que définis à la revendication 1.

12. A titre de médicament, les composés de formule (I) telle que définie à l'une quelconque des revendications 1 à 8, ainsi que les sels d'addition pharmaceutiquement acceptables.

13. A titre de médicament, les composés de formule (I) définis à la revendication 9.

14. Les compositions pharmaceutiques renfermant comme principe actif au moins l'un des médicaments définis à la revendication 12 ou 13.

15. A titre de produits intermédiaires, les composés de formules générales (IV), et (VI) telles que définies à la revendication 10.

## Patentansprüche

1. Verbindungen der Formel (I) 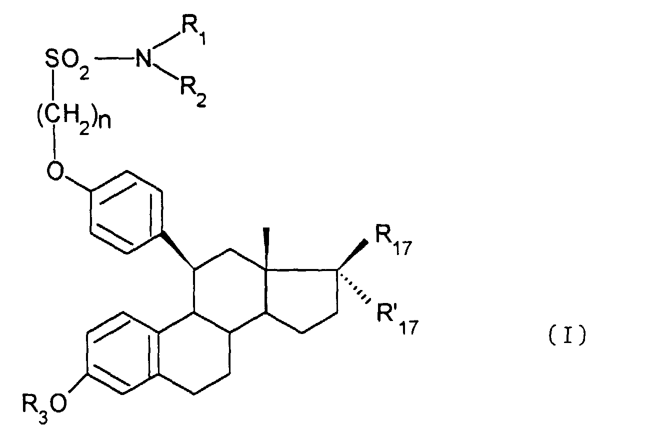 worin R₁₇ und R'₁₇ so definiert sind, daß
- entweder R₁₇ und R'₁₇ zusammen ein Keton bilden,
- oder R₁₇ einen Hydroxylrest oder einen Acyloxyrest mit höchstens 12 Kohlenstoffatomen bedeutet, R'₁₇ ein Wasserstoffatom, einen Alkyl-, Alkenyl- oder Alkinylrest mit höchstens 8 Kohlenstoffatomen bedeutet, wobei jeder der Reste gegebenenfalls substituiert ist,
- R₃ ein Wasserstoffatom, einen linearen, verzweigten oder cyclischen Alkylrest mit höchstens 8 Kohlenstoffatomen oder einen Acylrest mit höchstens 12 Kohlenstoffatomen bedeutet,
und worin R₁ und R₂, die gleich oder verschieden sind, ein Wasserstoffatom, einen linearen, verzweigten oder cyclischen Alkylrest mit 1 bis 8 Kohlenstoffatomen, der gegebenenfalls substituiert ist, einen Acylrest mit höchstens 12 Kohlenstoffatomen, einen Aryl- oder Aralkylrest bedeutet, wobei jeder dieser Reste gegebenenfalls substituiert ist, worin der Alkylrest höchstens 6 Kohlenstoffatome umfaßt und der Arylrest einen mono- oder polycyclischen Rest darstellt, der ein oder mehrere Heteroatom(e), ausgewählt aus den Atomen Sauerstoff, Stickstoff oder Schwefel, umfassen kann,
oder R₁ einen durch einen gegebenenfalls substituierten linearen, verzweigten oder cyclischen Alkylrest mit höchstens 8 Kohlenstoffatomen oder durch einen Aryl- oder Aralkylrest monosubstituierten Carbamoylrest bedeutet, wobei jeder dieser Reste gegebenenfalls substituiert ist, worin der Alkylrest höchstens 6 Kohlenstoffatome umfaßt und der Arylrest einen mono- oder polycyclischen Rest bedeutet, der ein oder mehrere Heteroatom(e), ausgewählt aus den Atomen Sauerstoff, Stickstoff und Schwefel, umfassen kann, und R₂ ein Wasserstoffatom bedeutet,
- oder R₁ und R₂ mit dem Stickstoffatom, an das sie gebunden sind, einen gesättigten 5- oder 6gliedrigen Stickstoffheterozyklus, umfassend gegebenenfalls ein zweites Heteroatom, ausgewählt aus den Atomen Stickstoff, Sauerstoff und Schwefel, gegebenenfalls substituiert durch einen Alkylrest mit 1 bis 4 Kohlenstoffatomen oder durch eine Oxogruppe, bilden,
- oder R₁ und R₂ einen Dialkylaminomethylenrest bilden, wobei jeder Alkylrest 1 bis 4 Kohlenstoffatome umfaßt, und n eine ganze Zahl von höchstens 18 ist,
sowie Additionssalze davon.

2. Verbindungen der allgemeinen Formel (I) nach Anspruch 1 der allgemeinen Formel (I') 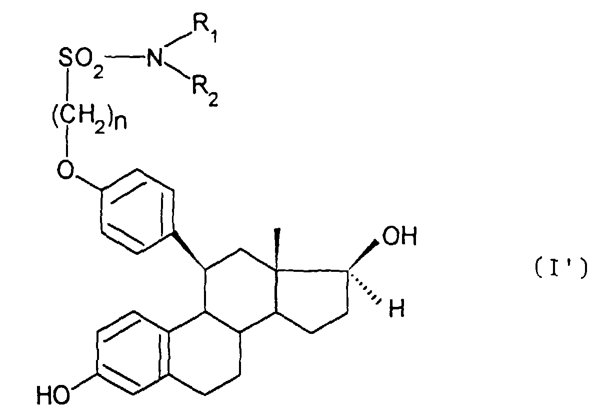 worin R₁ und R₂, die gleich oder verschieden sind, bedeuten: ein Wasserstoffatom, einen linearen, verzweigten oder cyclischen gegebenenfalls substituierten Alkylrest mit 1 bis 8 Kohlenstoffatomen, einen Acylrest mit höchstens 12 Kohlenstoffatomen oder einen gegebenenfalls substituierten Phenylrest, oder R₁ einen durch einen gegebenenfalls substituierten linearen, verzweigten oder cyclischen Alkylrest mit höchstens 8 Kohlenstoffatomen oder durch einen gegebenenfalls substituierten Phenylrest, monosubstituierten Carbamoylrest, bedeutet, und R₂ ein Wasserstoffatom bedeutet,
- oder R₁ und R₂ mit dem Stickstoffatom, an das sie gebunden sind, einen cyclischen Harnstoff vom Typ 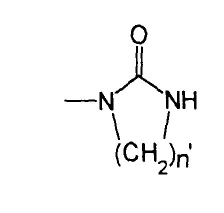 bilden, wobei n' 2 oder 3 bedeutet,
- oder R₁ und R₂ einen Dimethylaminomethylenrest bilden, n höchstens 7 bedeutet,
sowie die Additionssalze davon.

3. Verbindungen der Formel (I) nach Anspruch 1 oder 2, worin R₁₇ ein Hydroxylrest ist und R'₁₇ ein Wasserstoffatom bedeutet.

4. Verbindungen der Formel (I) nach Anspruch 1, 2 oder 3, worin n den Wert 5 oder 6 hat.

5. Verbindungen der Formel (I) nach einem der Ansprüche 1 bis 4, worin R₁ und R₂ ein Wasserstoffatom bedeuten.

6. Verbindungen der Formel (I) nach einem der Ansprüche 1 bis 4, worin R₁ und R₂ einen linearen, verzweigten oder cyclischen Alkylrest mit höchstens 8 Kohlenstoffatomen, der gegebenenfalls durch ein oder mehrere Halogenatom(e) substituiert ist, bedeuten.

7. Verbindungen der Formel (I) nach einem der Ansprüche 1 bis 4, worin R₁ ein Wasserstoffatom bedeutet, und R₂ einen linearen, verzweigten oder cyclischen Alkylrest mit höchstens 8 Kohlenstoffatomen bedeutet.

8. Verbindungen der Formel (I) nach einem der Ansprüche 1 bis 4, worin R₁ einen durch einen linearen, verzweigten oder cyclischen Alkylrest mit 1 bis 8 Kohlenstoffatomen oder durch einen Phenylrest monosubstituierten Carbamoylrest bedeutet, wobei jeder dieser Reste gegebenenfalls durch Halogenatome substituiert ist, und R₂ ein Wasserstoffatom bedeutet.

9. Verbindung der Formel (I) nach Anspruch 1, nämlich
- N-Butyl-5-[4-(3,17β-dihydroxyöstra-1,3,5(10)-trien-11β-yl)phenoxy]pentansulfonamid,
- N-Butyl-5-[4-(3,17β-dihydroxyöstra-1,3,5(10)-trien-11β-yl)phenoxy]-N-methylpentansulfonamid,
- 5-[4-(3,17β-Dihydroxyöstra-1,3,5(10)-trien-11β-yl)phenoxy]-N-(2,2,3,3,4,4,4-heptafluorbutyl)-N-methylpentansulfonamid.

10. Verfahren zur Herstellung von Verbindungen der Formel (I) nach Anspruch 1, dadurch **gekennzeichnet**, daß man eine Verbindung der Formel (II) 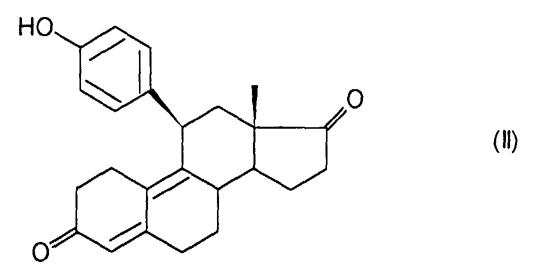 worin die Phenolfunktion gegebenenfalls geschützt ist,
a) entweder mit einem Halogenderivat der Formel (III)
X-(CH₂)ₙ-SO₂-NR_{A}R'_{A} (III)
umsetzt, worin X ein Halogenatom ist, n wie in Anspruch 1 definiert ist, R_{A} und R'_{A}, die gleich oder verschieden sind, ein Wasserstoffatom, einen gegebenenfalls substituierten linearen, verzweigten oder cyclischen Alkylrest mit 1 bis 8 Kohlenstoffatomen, einen Acylrest mit höchstens 12 Kohlenstoffatomen, einen Aryl- oder Aralkylrest, der gegebenenfalls substituiert ist, wie in Anspruch 1 definiert, bedeuten, mit der Maßgabe, daß mindestens einer der Substituenten R_{A} und R'_{A} kein Wasserstoffatom ist oder mit dem Stickstoff, an den sie gebunden sind, einen gesättigten 5- oder 6gliedrigen Stickstoffheterozyklus, der gegebenenfalls ein zweites Heteroatom, ausgewählt aus den Atomen Stickstoff, Sauerstoff und Schwefel, umfaßt und gegebenenfalls durch eine Alkylgruppe mit 1 bis 4 Kohlenstoffatomen substituiert ist, bilden, um eine Verbindung der Formel (IV) 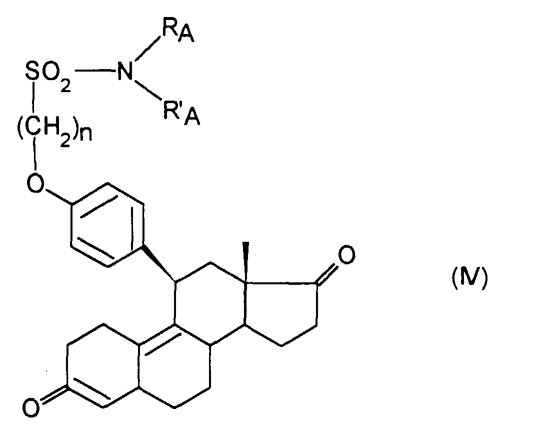 zu erhalten, worin n, R_{A} und R'_{A}, wie vorstehend definiert sind, man die Verbindung mit einem Aromatisierungsmittel für den Ring A umsetzt und gegebenenfalls den Hydroxylrest in Position 3 acyliert, um eine der Verbindung der Formel (I) entsprechende Verbindung der Formel (I_{A}) zu erhalten, worin R₁=R_{A} und R₂=R'_{A} 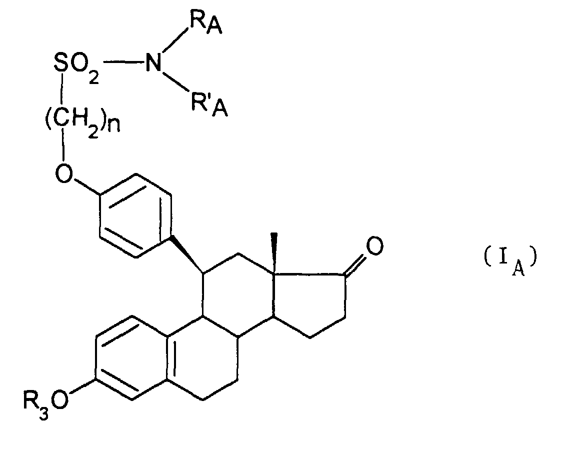 und worin n, R_{A} und R'_{A} wie vorstehend definiert sind und R₃ ein Wasserstoffatom oder eine Acylgruppe mit höchstens 12 Kohlenstoffatomen bedeutet, wobei man die Verbindung der Formel (I_{A}) gegebenenfalls und notwendigenfalls einer oder mehrerer der folgenden Reaktionen in geeigneter Reihenfolge unterwirft:
- Reduktion der Ketonfunktion in Position 17
- Addition eines Metallkomplexes der Formel (X):
M-R'₁₇ₐ (X)
an die Ketonfunktion in Position 17, worin M ein Metallatom bedeutet und R'₁₇ₐ wie R'₁₇, ausgenommen ein Wasserstoffatom, definiert ist,
- selektive Acylierung in Position 17, wenn R₁₇ ein Hydroxylrest ist,
- Alkylierung oder Acylierung des Hydroxylrests in Position 3
- Verseifung, wenn R₃ eine Acylfunktion bedeutet
- gegebenenfalls Salzbildung durch eine Säure oder eine Base
B) oder mit einem Aromatisierungsmittel für den Zyklus A umsetzt und den Hydroxylrest in Position 3 mit einer Schutzgruppe versieht, dann selektiv die Phenolschutzgruppe in Position 11 abspaltet, um eine Verbindung der Formel (V) 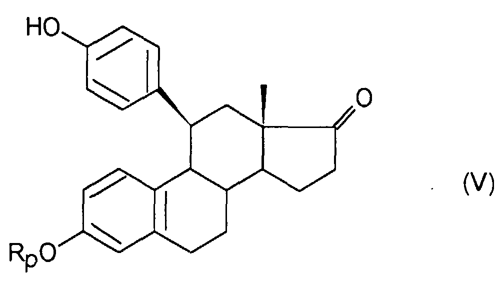 zu erhalten, worin Rₚ eine Schutzgruppe bedeutet, man die Verbindung mit einer Verbindung der Formel (III')
X-(CH₂)ₙ-SO₂-N=CH-N(Alk₁)(Alk₂) (III')
umsetzt, wobei X ein Halogenatom ist, (Alk₁) und (Alk₂) Alkylreste mit 1 bis 4 Kohlenstoffatomen sind und n höchstens 18 bedeutet, anschließend das gebildete Imin hydrolysiert, um eine Verbindung der Formel (VI) 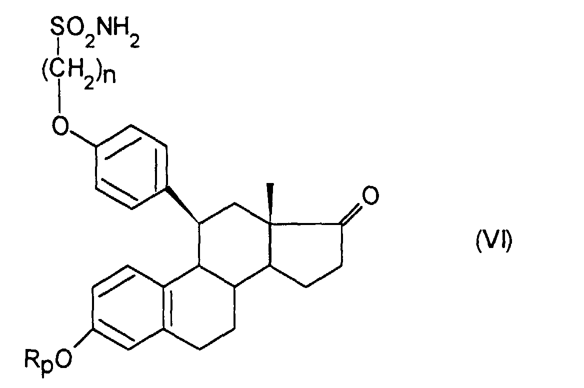 zu erhalten, worin n und Rₚ wie vorstehend definiert sind, die gegebenenfalls entsprechend der Natur der Gruppierung Rₚ einer Verbindung der Formel (I) entsprechen kann, wobei man die Verbindung der Formel (VI) gegebenenfalls und notwendigenfalls einer oder mehrerer der nachstehenden Reaktionen in geeigneter Reihenfolge unterwirft:
- Eliminierungsreaktion der Schutzgruppe Rₚ
- Reduktion der Ketonfunktion in Position 17
- Addition an die Ketonfunktion in Position 17 eines metallischen Komplexes der Formel (X)
M-R'₁₇ₐ (X)
wie vorstehend definiert,
- selektive Acylierung in Position 17, wenn R₁₇ ein Hydroxylrest ist,
- Alkylierung oder Acylierung des Hydroxylrests in Position 3
- Einwirkung eines Halogenids der Formel (VII)
R_{B}-X (VII)
worin R_{B} ein gegebenenfalls substituierter Alkylrest mit 1 bis 4 Kohlenstoffatomen oder ein Acylrest mit höchstens 12 Kohlenstoffatomen ist und X ein Halogenatom bedeutet, um die der Verbindung der Formel (I) entsprechende Verbindung der Formel (I_{B}) zu erhalten, worin R₁=H und R₂=R_{B} 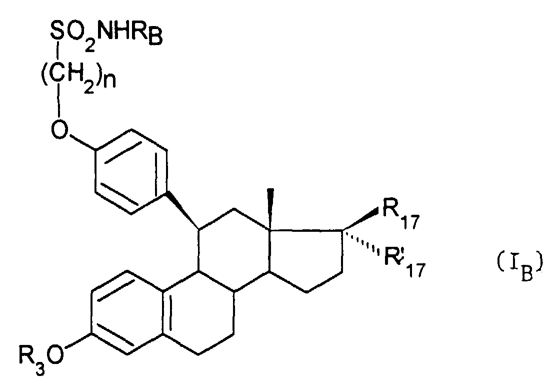 und worin n, R₁₇, R'₁₇ und R₃ wie in Anspruch 1 definiert sind,
- Einwirkung eines Isocyanats der Formel (VIII)
R_{C}-NCO (VIII)
worin R_{C} einen linearen, verzweigten oder cyclischen Alkylrest mit höchstens 8 Kohlenstoffatomen, Aryl oder Aralkyl wie in Anspruch 1 definiert, bedeutet, wobei jeder der Reste gegebenenfalls substituiert ist, um die der Verbindung der Formel (I) entsprechende Verbindung der Formel (I_{C}) zu erhalten, worin R₁ einen monosubstituierten Carbamoylrest und R₂ ein Wasserstoffatom bedeutet, 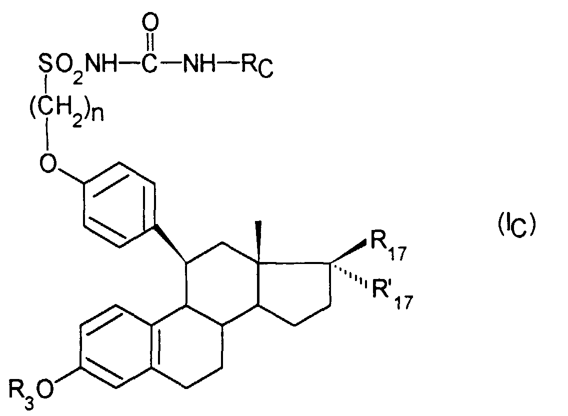 und worin n, R₁₇, R'₁₇ und R₃ wie in Anspruch 1 definiert sind,
- Einwirkung einer Verbindung der Formel (IX)
(Alk₃O)(Alk₄O)CH-N(Alk₁)(Alk₂) (IX)
worin Alk₁, Alk₂, Alk₃ und Alk₄ Alkylreste mit 1 bis 4 Kohlenstoffatomen sind, um eine der Verbindung der Formel (I) entsprechende Verbindung der Formel (I_{D}) zu erhalten, worin R₁ und R₂ eine Dialkylaminomethylengruppe bilden 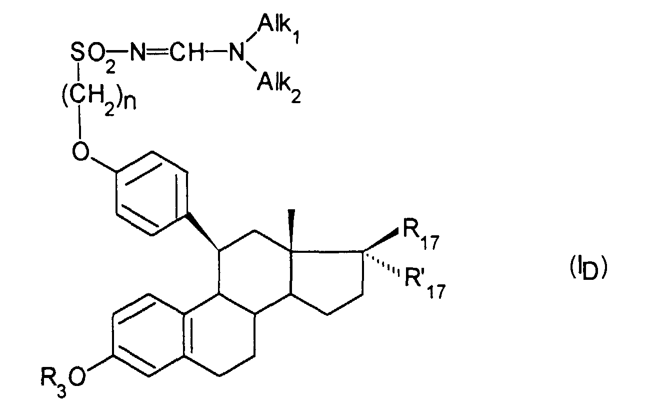 und worin n, R₁₇, R'₁₇ und R₃ wie in Anspruch 1 definiert sind,
- Alkylierung oder Acylierung der Verbindung der Formel (I_{B}) durch ein Halogenid der Formel (VII), um ein dialkyliertes, diacyliertes oder alkylacyliertes entsprechendes Sulfonamid zu erhalten,
- Cyclisierungsreaktion der Verbindung der Formel (I_{C}), wenn R_{c} ein Rest -(CH₂)_{n'}-Hal ist, wobei n' 2 oder 3 ist und Hal ein Halogenatom bedeutet, um die der Verbindung der Formel (I) entsprechende Verbindung der Formel (I'_{C}) zu erhalten, worin R₁ und R₂ zusammen mit dem Stickstoff, an den sie gebunden sind, einen cyclischen Harnstoff des Typs 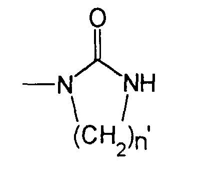 bilden,
- gegebenenfalls Salzbildung durch eine Säure oder eine Base.

11. Verfahren zur Herstellung von Verbindungen der Formel (I) nach Anspruch 1, dadurch **gekennzeichnet**, daß man das Verfahren analog Anspruch 10 durchführt, und daß man als Ausgangsverbindung eine der Verbindung der Formel (II) entprechende Verbindung, wie in Anspruch 10 definiert, die in Position 17 die Substituenten R₁₇ und R'₁₇, wie in Anspruch 1 definiert, trägt, verwendet.

12. Verbindungen der Formel (I) nach einem der Ansprüche 1 bis 8 sowie ihre pharmazeutisch verträglichen Additionssalze als Medikament.

13. Verbindungen der Formel (I) nach Anspruch 9 als Medikament.

14. Pharmazeutische Zusammensetzungen, umfassend als Wirkstoff mindestens eines der in Anspruch 12 oder 13 definierten Medikamente.

15. Verbindungen der allgemeinen Formeln (IV) und (VI), wie in Anspruch 10 definiert, als Zwischenprodukte.

## Claims

1. The compounds of formula (I): 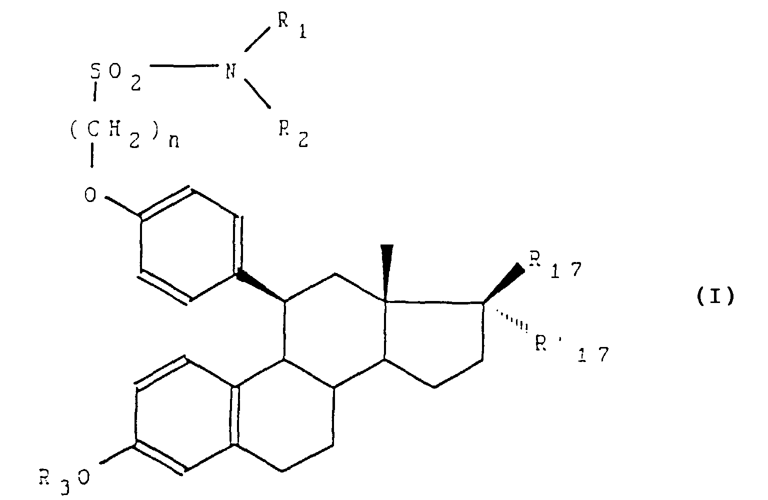 in which R₁₇ and R'₁₇ are such that:
- either R₁₇ and R'₁₇ together form a ketone
- or R₁₇ represents a hydroxyl radical, or an acyloxy radical having at most 12 carbon atoms and R'₁₇ represents a hydrogen atom, an alkyl, alkenyl or alkynyl radical having at most 8 carbon atoms, each of these radicals being optionally substituted,
- R₃ represents a hydrogen atom, a linear, branched or cyclic alkyl radical having at most 8 carbon atoms or an acyl radical having at most 12 carbon atoms,
and in which R₁ and R₂, identical or different, represent a hydrogen atom, a linear, branched or cyclic alkyl radical containing 1 to 8 carbon atoms, optionally substituted, an acyl radical having at most 12 carbon atoms, an aryl or aralkyl radical, each of these radicals being optionally substituted, in which the alkyl radical contains at most 6 carbon atoms and the aryl radical represents a mono or polycyclic radical which can contain one or more heteroatoms chosen from oxygen, nitrogen and sulphur atoms,
or R₁ represents a carbamoyl radical monosubstituted by a linear, branched or cyclic alkyl radical having at most 8 carbon atoms, optionally substituted, or by an aryl or aralkyl radical, each of these radicals being optionally substituted, in which the alkyl radical contains at most 6 carbon atoms and the aryl radical represents a mono or polycyclic radical which can contain one or more heteroatoms chosen from oxygen, nitrogen and sulphur atoms and R₂ represents a hydrogen atom,
- or R₁ and R₂ form with the nitrogen atom to which they are linked a saturated nitrogenous heterocycle with 5 or 6 members optionally containing a second heteroatom, chosen from nitrogen, oxygen and sulphur atoms, optionally substituted by an alkyl radical having 1 to 4 carbon atoms, or by an oxo group,
- or R₁ and R₂ form a dialkylaminomethylene radical, each alkyl containing 1 to 4 carbon atoms,
n is an integer at most equal to 18,
as well as the addition salts of the aforementioned.

2. The compounds of general formula (I) as defined in claim 1, corresponding to general formula (I'): 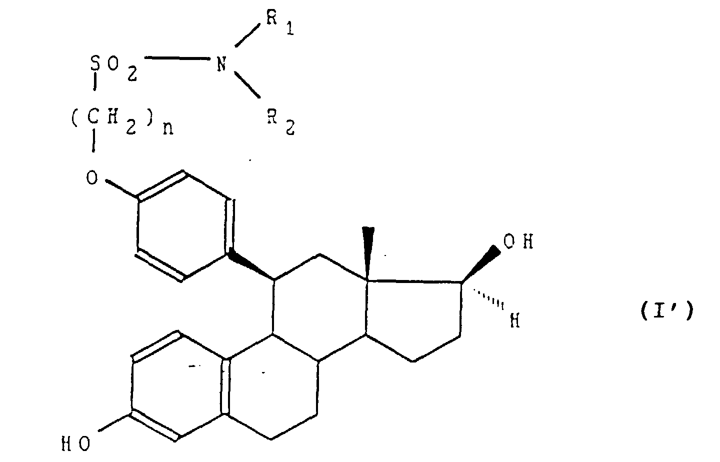 in which R₁ and R₂, identical or different, represent:
a hydrogen atom, a linear, branched or cyclic alkyl radical containing 1 to 8 carbon atoms, optionally substituted, an acyl radical having at most 12 carbon atoms, or an optionally substituted phenyl radical,
or R₁ represents a carbamoyl radical monosubstituted by an optionally substituted linear, branched or cyclic alkyl radical having at most 8 carbon atoms, or by an optionally substituted phenyl radical, and R₂ represents a hydrogen atom,
or R₁ and R₂ form with the nitrogen atom to which they are linked, a cyclic urea of the type 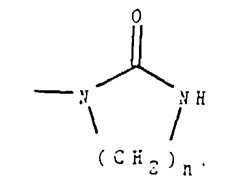
n' being equal to 2 or 3,
- or R₁ and R₂ form a dimethylaminomethylene radical, n is equal at most to 7,
as well as the addition salts of the aforementioned.

3. The compounds of formula (I) as defined in claim 1 or 2, in which R₁₇ is a hydroxyl radical and R'₁₇ represents a hydrogen atom.

4. The compounds of formula (I) as defined in claim 1, 2 or 3 in which n is equal to 5 or 6.

5. The compounds of formula (I) as defined in any one of claims 1 to 4, in which R₁ and R₂ represent a hydrogen atom.

6. The compounds of formula (I) as defined in any one of claims 1 to 4, in which R₁ and R₂ represent a linear, branched or cyclic alkyl radical having at most 8 carbon atoms optionally substituted by one or more halogen atoms.

7. The compounds of formula (I) as defined in any one of claims 1 to 4, in which R₁ represents a hydrogen atom and R₂ represents a linear, branched or cyclic alkyl radical having at most 8 carbon atoms.

8. The compounds of formula (I) as defined in any one of claims 1 to 4, in which R₁ represents a carbamoyl radical monosubstituted by a linear, branched or cyclic alkyl radical containing 1 to 8 carbon atoms, or by a phenyl radical, each of these radicals being optionally substituted by halogen atoms and R₂ represents a hydrogen atom.

9. The compounds of formula (I) as defined in claim 1 of which the names follow:
N-butyl-5-[4-(3,17beta-dihydroxy-estra-1,3,5(10)-trien-11beta-yl) phenoxy]-pentanesulphonamide,
N-butyl 5-[4-(3,17beta-dihydroxy-estra-1,3,5(10)-trien-11beta-yl) phenoxy]-N-methyl-pentanesulphonamide,
5-[4-(3,17beta-dihydroxy-estra-1,3,5(10)-trien-11beta-yl) phenoxy]-N-(2,2,3,3,4,4,4-heptafluorobutyl)-N-methylpentanesulphonamide.

10. Preparation process for the compounds of formula (I) as defined in claim 1, characterized in that a compound of formula (II): 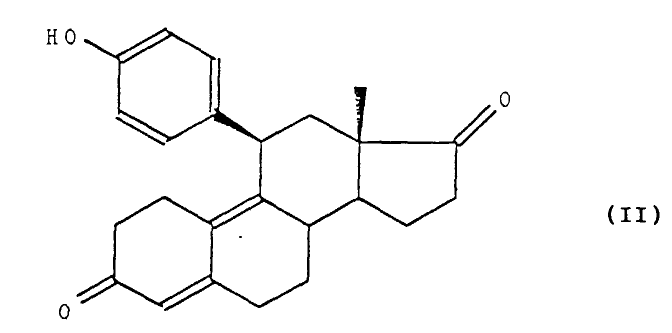 in which the phenol function is, if appropriate, protected, is subjected
**A) Either** to the action of a halogenated derivative of formula (III):
X-(CH₂)ₙ-SO₂-NR_{A}R'_{A} (III)
in which X is a halogen atom, n has the meaning indicated in claim 1, R_{A} and R'_{A}, identical or different, represent a hydrogen atom, an optionally substituted linear, branched or cyclic alkyl radical containing 1 to 8 carbon atoms, an acyl radical having at most 12 carbon atoms, an optionally substituted aryl or aralkyl radical, as defined in claim 1, it being understood that at least one of the substituents R_{A} and R'_{A} is not a hydrogen atom, or form with the nitrogen to which they are linked a saturated nitrogenous heteroatom with 5 or 6 members optionally containing a second heteroatom chosen from nitrogen, oxygen and sulphur atoms and optionally substituted by an alkyl group having 1 to 4 carbon atoms, in order to obtain a product of formula (IV): 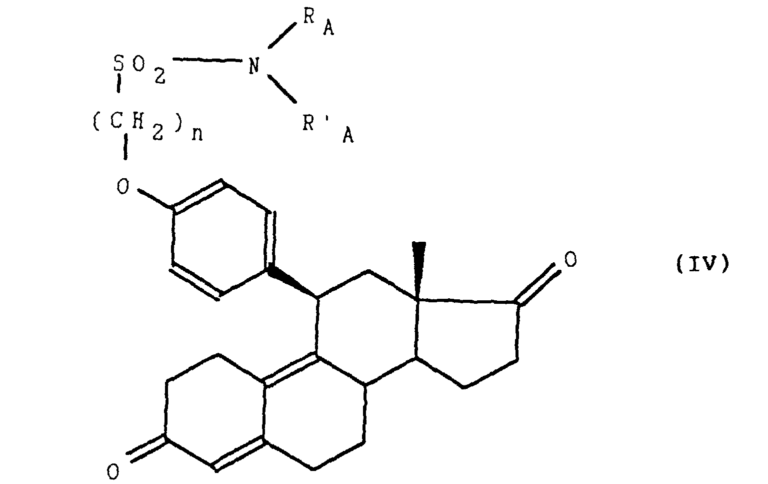 in which n, R_{A} and R'_{A} have the same meaning as previously, which is subjected to the action of an aromatization agent of ring A, and optionally of an acylation agent of the hydroxyl radical in position 3, in order to obtain a product of formula (I_{A}), corresponding to the product of formula (I), in which R₁=R_{A} and R₂=R'_{A}, 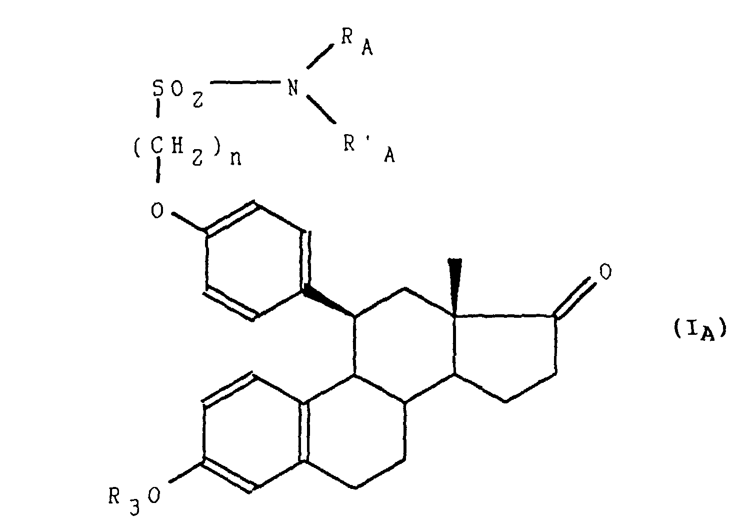 and in which n, R_{A} and R'_{A} have the same meaning as previously, and R₃ represents a hydrogen atom or an acyl group having at most 12 carbon atoms, which product of formula (I_{A}) is subjected, if desired and if necessary, to one or more of the following reactions, in an appropriate order:
- reduction of the ketone function in position 17
- addition on the ketone function in position 17 of a metal complex of formula (X):
M-R'₁₇ₐ (X)
in which M represents a metal atom and R'₁₇ₐ has the same meaning as R'₁₇ with the exception of a hydrogen atom,
- selective acylation in position 17 when R₁₇ is a hydroxyl,
- alkylation or acylation of the hydroxyl radical in position 3,
- saponification when R₃ represents an acyl function,
- optional salification by an acid or a base
**B) Or** to the action of an aromatization agent of the A ring and to a protection reaction of the hydroxyl radical in position 3, then to a selective elimination reaction of the protective group of the phenol in position 11, in order to obtain a product of formula (V): 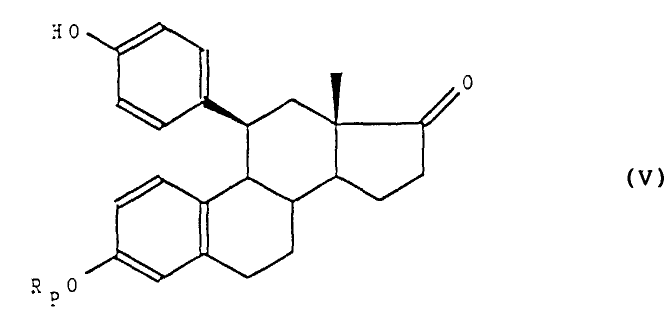 in which Rₚ represents a protective group, which is subjected to the action of a compound of formula (III'):
X-(CH₂)ₙ-SO₂-N=CH-N(Alk₁)(Alk₂) (III')
X being a halogen atom, (Alk₁) and (Alk₂) being alkyl groups having 1 to 4 carbon atoms and n is equal at most to 18, then to a hydrolysis reaction of the imine formed, in order to obtain a compound of formula (VI): 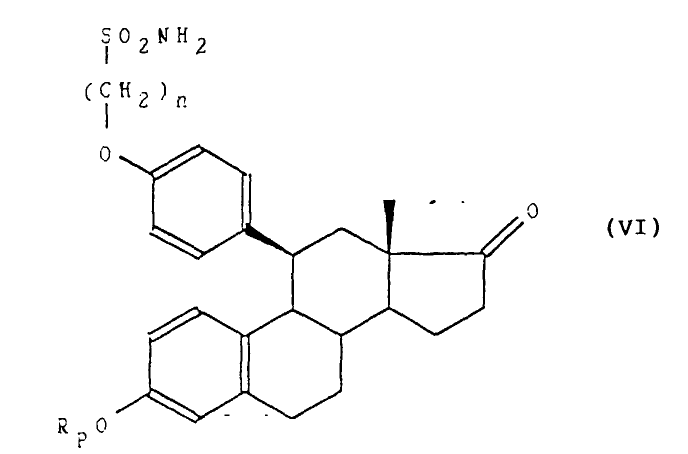 and in which n and Rₚ have the same meaning as previously, being able if appropriate, according to the nature of the Rₚ group, to correspond to a product of formula (I), which product of formula (VI) is subjected, if desired and if necessary, to one or more of the following reactions, in an appropriate order:
- elimination reaction of the Rₚ protective group
- reduction of the ketone function in position 17
- addition on the ketone function in position 17 of a metal complex of formula (X):
M-R'₁₇ₐ (X)
as defined previously,
- selective acylation in position 17 when R₁₇ is a hydroxyl
- alkylation or acylation of the hydroxyl radical in position 3
- action of a halide of formula (VII)
R_{B}-X (VII)
in which R_{B} is an optionally substituted alkyl radical having 1 to 4 carbon atoms, or an acyl radical having at most 12 carbon atoms and X represents a halogen atom in order to obtain the compound of formula (I_{B}) corresponding to the product of formula (I) in which R₁=H and R₂=R_{B}, 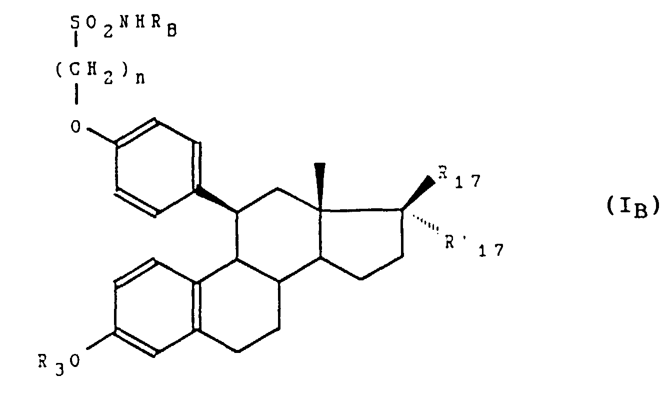 and in which n, R₁₇, R'₁₇ and R₃ have the meanings indicated in claim 1,
- action of an isocyanate of formula (VIII):
R_{C}-NCO (VIII)
in which R_{C} represents a linear, branched or cyclic alkyl radical having at most 8 carbon atoms, an aryl or aralkyl radical as defined in claim 1, each of the radicals being optionally substituted in order to obtain the compound of formula (I_{C}), corresponding to the product of formula (I) in which R₁ represents a mono-substituted carbamoyl radical and R₂ a hydrogen atom, 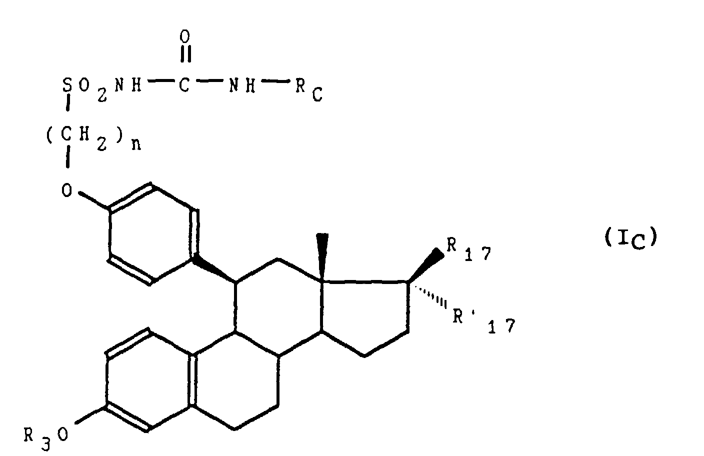 and in which n, R₁₇, R'₁₇ and R₃ have the meanings indicated in claim 1,
- action of a compound of formula (IX):
(Alk₃O)(Alk₄O)CH-N(Alk₁)(Alk₂) (IX)
in which Alk₁, Alk₂, Alk₃ and Alk₄ are alkyl radicals having 1 to 4 carbon atoms, in order to obtain the compound of formula (I_{D}), corresponding to the product of formula (I) in which R₁ and R₂ form a dialkylaminomethylene group, 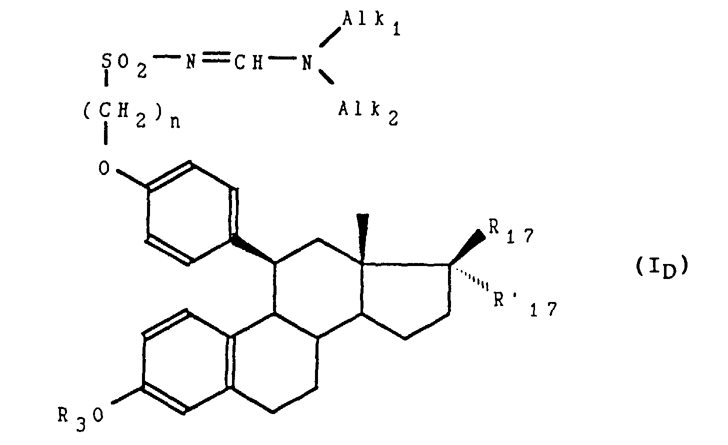 and in which n, R₁₇, R'₁₇ and R₃ have the meanings indicated in claim 1,
- alkylation or acylation of the product of formula (I_{B}), by the action of a halide of formula (VII), in order to obtain a corresponding dialkylated, diacylated or alkylacylated sulphonamide,
- cyclization reaction of the product of formula (I_{C}), when R_{C} is a -(CH₂)ₙ'-Hal radical, n' being equal to 2 or 3 and Hal represents a halogen atom, in order to obtain the product of formula (I'_{C}) corresponding to the product of formula (I) in which R₁ and R₂ form with the nitrogen to which they are linked a cyclic urea of the type: 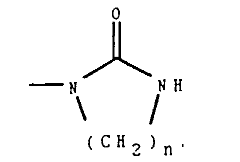
- optional salification by an acid or a base.

11. Preparation process for the products of formula (I) as defined in claim 1, characterized in that the process is carried out in a similar way to claim 10 and in that a compound is used at the start corresponding to the compound of formula (II) as defined in claim 10 containing in position 17 the R₁₇ and R'₁₇ substituents as defined in claim 1.

12. As medicaments, the compounds of formula (I) as defined in any one of claims 1 to 8, as well as the pharmaceutically acceptable addition salts.

13. As medicaments, the compounds of formula (I) defined in claim 9.

14. The pharmaceutical compositions containing as active ingredient at least one of the medicaments defined in claim 12 or 13.

15. As intermediate products, the compounds of general formulae (IV) and (VI) as defined in claim 10.
